# EUROPEAN PATENT SPECIFICATION

(11) **EP 4 732 013 B1**
(45) Date of publication and mention of the grant of the patent: **03.06.2026**
(21) Application number: 24783374.2
(22) Date of filing: 26.09.2024
(51) Int. Cl.: G01N 33/575, G01N 33/57505

(54) **METHODS, REAGENTS AND KITS FOR DETECTING MINIMAL/MEASURABLE DISEASE IN T-CELL ACUTE LYMPHOBLASTIC LEUKEMIA (T-ALL) AND T-CELL LYMPHOBLASTIC LYMPHOMA (T-LBL)**
VERFAHREN, REAGENZIEN UND KITS ZUM NACHWEIS VON MINIMALEN/MESSBAREN ERKRANKUNGEN BEI AKUTER LYMPHOBLASTISCHER T-ZELL-LEUKÄMIE (T-ALL) UND LYMPHOBLASTISCHEM T-ZELL-LYMPHOM (T-LBL)
PROCÉDÉS, RÉACTIFS ET KITS POUR DÉTECTER UNE MALADIE MINIMALE/MÉARISABLE DANS LA LEUCÉMIE LYMPHOBLASTIQUE AIGUË À LYMPHOCYTES T (T-ALL) ET LE LYMPHOME LYMPHOBLASTIQUE À LYMPHOCYTES T (T-LBL)

(30) Priority: 26.09.2023 EP 23199775
(43) Date of publication of application: 29.04.2026
(73) Proprietor: Stichting EuroFlow, 7202 AG Zutphen (NL)
(72) Inventor: ORFAO DE MATOS CORREIA E VALE, José Alberto, 37008 Salamanca (ES); SZCZEPANSKI, Tomasz, 40-055 Katowice (PL); SEDEK, Lukasz, 40-055 Katowice (PL); SORIANO RODRIGUEZ, Beatriz, 37008 Salamanca (ES); LHERMITTE, Ludovic Bernard Simon, 75015 Paris (FR); BRÜGGEMANN, Monika Ursula Helga, 24105 Kiel (DE); KOHLSCHEEN, Saskia, 24105 Kiel (DE); KALINA, Tomás, 116 36 Praha (CZ); REITEROVÁ, Michaela, 116 36 Praha (CZ); NIERKENS, Stefan, 3584 CS Utrecht (NL); GAIPA, Giuseppe, 20900 Monza (IT); DE SA FERREIRA FACIO, Cristiane, 21941-912 Rio de Janeiro (BR); ALMEIDA PARRA, Julia Maria, 37008 Salamanca (ES); VAN DONGEN, Jacobus Johannes Maria, 37008 Salamanca (ES)
(74) Representative: V.O.
(86) International application number: PCT/NL2024/050525
(87) International publication number: WO 2025/071406

(56) References cited:
- WO-A1-2022/236181
- PRASHANT R TEMBHARE ET AL: "Eleven-marker 10-color flow cytometric assessment of measurable residual disease for T-cell acute lymphoblastic leukemia using an approach of exclusion", CYTOMETRY PART B CLINICAL CYTOMETRY, WILEY-LISS, HOBOKEN, NJ, US, vol. 100, no. 4, 19 August 2020 (2020-08-19), pages 421 - 433, XP072335200, ISSN: 1552-4949, DOI: 10.1002/CYTO.B.21939
- JOSEPH A DIGIUSEPPE ET AL: "Applications of Flow Cytometric Immunophenotyping in the Diagnosis and Posttreatment Monitoring of B and T Lymphoblastic Leukemia/Lymphoma", CYTOMETRY PART B CLINICAL CYTOMETRY, WILEY-LISS, HOBOKEN, NJ, US, vol. 96, no. 4, 24 June 2019 (2019-06-24), pages 256 - 265, XP072339182, ISSN: 1552-4949, DOI: 10.1002/CYTO.B.21833
- DENYS B ET AL: "Improved flow cytometric detection of minimal residual disease in childhood acute lymphoblastic leukemia", BLOOD CANCER JOURNAL, NATURE PUBLISHING GROUP UK, LONDON, vol. 27, no. 3, 16 August 2012 (2012-08-16), pages 635 - 641, XP037786138, ISSN: 0887-6924, [retrieved on 20120816], DOI: 10.1038/LEU.2012.231

## Description

The invention relates to the field of leukemia/lymphoma diagnosis, more specifically to the detection of minimal numbers of leukemia/lymphoma cells in bone marrow, blood and other fluids and tissues from patients with T-cell acute lymphoblastic leukemia and T-cell lymphoblastic lymphoma (T-ALL/T-LBL). The invention can be used to monitor minimal/measurable residual disease (MRD) during and after therapy with chemotherapeutic agents, immunotherapy or combinations thereof.

In the last decades, treatment of T-ALL/T-LBL has evolved substantially with progressively higher rates of complete remission (CR) both in children and, to a lesser extent, also in adults [1]. However, still many patients that reach CR, early or later on during or after therapy show disease progression and relapse/recurrence [2]. Previous studies have demonstrated that such relapses are due to the persistence of leukemia/lymphoma cells at levels below the detection sensitivity of the conventional techniques used to evaluate response to therapy, such as cytomorphology. This is because the sensitivity of cytomorphology reaches only levels of detection of leukemia/lymphoma cells of 1-5% (10⁻²) among the whole sample cellularity, pointing out the need for more sensitive techniques. The availability of techniques with a higher sensitivity provides a better insight in the reduction of tumor mass during induction therapy and further clearance of the residual T-ALL/T-LBL leukemia/lymphoma cells with their potential eradication with the subsequent doses of consolidation and/or maintenance therapy.

Since the 90's, several different molecular and immunophenotyping methods have been developed for the detection of minimal numbers of T-ALL/T-LBL leukemia/lymphoma cells, at levels below the sensitivity of conventional cytomorphology (e.g., <10⁻³) [3,4]. Based on these approaches, the persistence of T-ALL/T-LBL leukemia/lymphoma cells at MRD levels has been demonstrated to be a robust marker for the evaluation of the quality of CR, with a significant impact on subsequent patient outcome [4]. Because of this, more recently, MRD has been further introduced in daily clinical practice as a new prognostic factor for 1) re-stratification of patient risk of undergoing disease recurrence/relapse, and 2) for making decisions about the optimal (subsequent) treatment strategy. Thereafter, subsequent use of MRD-guided treatment protocols has contributed to further improve patient outcome, due to even deeper responses to therapy and better long-term disease control [5]. However, even with the use of current molecular and conventional ≥4 flow cytometry MRD approaches that reach sensitivities of <10⁻⁴, still a fraction of patients with undetected MRD suffer from disease recurrence, pointing out the need for even more sensitive MRD techniques [6].

Among the immunophenotypic-based MRD methods, multiparametric flow cytometry immunophenotyping for detection of leukemia/lymphoma-associated immunophenotypes, has become the method of choice. At present, it is well-established that the sensitivity of flow cytometry MRD methods depends on both 1) the combination of markers used to specifically identify the residual (e.g., T-ALL/T-LBL) leukemia/lymphoma cells and to distinguish them from all other (normal and reactive) cells coexisting in the same sample, and 2) the total number of cells investigated. However, up to now, the EuroFlow Consortium and other groups working in the MRD field failed to provide unique reagent compositions for monitoring MRD in T-ALL/T-LBL by flow cytometry with sufficiently high sensitivity.

Early immunophenotypic MRD studies in T-ALL/T-LBL were based on 2- and 3-color conventional flow cytometry, where ≥2 aliquots of the same sample were stained in parallel with different combinations of MRD markers. Initially, Orfao et al. proposed in 1994 the use of the CD1a, CD2, surface membrane (sm)CD3, cytoplasmic (cy)CD3, CD4, CD5, CD8 and TCRαB T-cell associated markers, the CD38 and CD71 precursor/activation-associated markers, and the CD13 and CD33 myeloid and stem cell-associated markers arranged in 3-color combinations of fluorochrome-conjugated markers analyzed by flow cytometry for MRD detection in T-ALL/T-LBL [8]. However, they failed in specifying how to combine these markers in the 3-color stainings. One year later, Campana and Pui proposed the use of a single 2-color combination with Terminal deoxynucleotidyl transferase (TdT) and cyCD3 for MRD detection in T-ALL/T-LBL [9]. However, the above two early panels, allowed detection of aberrant or rare leukemia/lymphoma-associated phenotypes in only a fraction of T-ALL/T-LBL leukemia/lymphoma blasts, since a significant percentage of blasts within individual patients did not express the aberrant phenotypes identifiable with the individual antibody reagent combination proposed [8]. This translated into a limited applicability and/or sensitivity of both panels. In order to increase the applicability of conventional flow cytometry for MRD detection in T-ALL/T-LBL in these settings, Campana and Coustan-Smith subsequently proposed an additional 2-color tube to be added to the former TdT + cyCD3 marker combination, consisting of CD34 + cyCD3, with limited impact on the percentage of cases in which an aberrant phenotype could be identified as only a fraction of blast cells from a part of T-ALL/T-LBL patients express the CD34 marker [10].

Subsequent proposals of improved antibody reagent combinations, systematically included at least one marker in common among the multiple tubes used to stain different aliquots of the same sample for MRD purposes, for a greater consistency on the identification of those cell compartments in the sample containing the T-ALL/T-LBL cells and their discrimination from other (e.g., myeloid) normal cells coexisting in the same sample. Thus, Porwit-MacDonald et al. proposed the parallel staining of up to 5 different sample aliquots, each with unique combination of 3 markers, of which one was shared in all five stained aliquots (i.e., CD7) and the other two varied among said aliquots: 1) CD5 and smCD3, 2) CD4 and CD8, 3) CD2 and smCD3, 4) TdT and cyCD3 and 5) CD38 and CD34. MRD blasts were systematically identified based on 1) co-expression of CD7 and immature T-cell markers (e.g., TdT, CD34) or 2) expression of CD7 in the absence of mature T-cell-associated antigens (e.g., smCD3, CD5, CD4-only, CD8-only, CD2) [11]. In turn, Dworzak et al. proposed a parallel staining of two 3-color marker combinations in which cyCD3 and smCD3 were always stained in common in both tubes, together with either TdT or CD7 [12]. The same year, Coustan-Smith et al. added CD5 in their previous 2-color panel described above, proposing the staining for TdT CD5 cyCD3 in tube 1 and CD34 CD5 cyCD3 in tube 2 [13]. This same panel was adopted later on by Bjorklund et al. [14].

Despite these improvements, all 3-color antibody combinations described above, still displayed several important limitations: 1) they could not identify aberrant phenotypes present in the whole leukemia/lymphoma cell population in a substantial proportion of T-ALL/T-LBL cases, and 2) they showed limited sensitivity due to the presence of minimal numbers of normal cells displaying overlapping phenotypes with T-ALL/T-LBL cells. Among other overlapping phenotypes between T-ALL/T-LBL cells and normal residual cells, these included tumor phenotypes overlapping with small populations of 1) cyCD3⁺ smCD3⁻ CD7⁺ NK-cells, 2) CD7⁺ CD5⁺ smCD3⁻ NK-cells, 3) CD7⁺ CD4-CD8⁻T-cells and NK-cells, 4) CD7⁺ CD2⁻ smCD3⁻ NK-cells, 5) TdT⁻ CD7⁺ cyCD3⁺ smCD3⁻ NK-cells and smCD3⁺ T-cells, 6) CD7⁺ CD38⁺ CD34⁺ normal hematopoietic precursors, 7) TdT⁻ CD5^{-/lo} cyCD3⁺ smCD3⁻ NK-cells and 8) TdT⁻ CD34- cyCD3⁺ T-cells and NK-cells.

Because of these limitations, ≥4-color combinations of fluorochrome-conjugated antibody reagents were subsequently composed, proposed and tested even by the same authors. In this regard, Vidriales et al. proposed a panel consisting of six 4-color tubes in which CD7 was systematically used in common in all tubes like in the earlier Porwit-MacDonald et al. panel [11], together with an immaturity marker (CD34, cyCD3 and/or TdT) and either a mature T-cell marker like CD5, CD2 and CD4+CD8, or the CD13 or the CD33 myeloid and stem cell-associated markers [15]. Immediately after, Dworzak et al. proposed a 3-tube, 4-color antibody panel for MRD detection in T-ALL/T-LBL, where some of the above markers were combined with a new marker (CD99) used in their proposal for the first time [16]. In the new Dworzak et al. panel, smCD3 and CD7 (instead of cyCD3) were now used in common in all 3 tubes, in combination with cyCD3 and CD99 or TdT in the first two tubes, respectively, or in combination with CD99 and CD5 in the remaining third tube [16]. Despite taking advantage of adding an extra marker and an extra tube, the same limitations regarding sensitivity remained as low levels of CD99 can be detected in normal T-cells and in a substantial proportion of T-ALL/T-LBL patients the blasts might show, at least in part, absence or low CD99 expression.

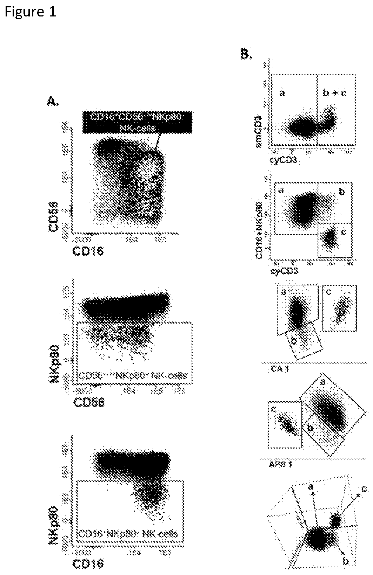

A major limitation shared by all above panels was due to the presence of a small population of cyCD3⁺ smCD3⁻ CD7⁺ normal/residual NK-cells (Figure 1A), in addition to another subset of normal CD7⁺ CD34⁺ and TdT⁺ CD34⁺ hematopoietic precursors among all normal leukocytes present in e.g., human bone marrow. In addition, all the above antibody panels lacked on a marker for accurate identification of the major populations of maturing neutrophils, monocytic cells, mature B-, T- and NK-cells and nucleated red cells and their discrimination from the normal and malignant precursor cells, which allowed at the same time the use of a reliable and robust denominator in calculating the exact percentage of residual T-ALL/T-LBL cells present in the sample. To achieve this latter goal, the CD45 marker was introduced in the subsequent MRD panels, as described below.

In the following decade, 3- and 4-color variant combinations of the same markers were used by Enein et al. [17], Salari et al. [18] and van Wering et al. [19], respectively, without overcoming the above mentioned limitations of MRD detection by conventional flow cytometry. In this same period, several other authors proposed panels of ≥3 fluorochrome-conjugated antibody reagent combinations devoted to MRD monitoring in T-ALL/T-LBL, which included between 6 and 10 markers per antibody combination (i.e., per tube). In an attempt to overcome the limitations of the above ≤4 color panels, and in order 1) to exclude mature NK-cells as well as normal precursors, and 2) to provide a reliable denominator for calculating the relative percentage of MRD cells, Denys et al. [20] and Wood et al. [21] proposed 6- and 9-color antibody panels consisting of 2 different tubes in which CD45, and the CD56 and CD16 NK-cell markers (with the same or different fluorochromes) were added to combinations of between 4 and 7 different markers that had been previously reported and used in the literature for MRD detection in T-ALL/T-LBL: 1) CD2 CD5 CD7 smCD3 or CD99 cyCD3 CD7 smCD3 [20]; and 2) cyCD3 CD7 CD5 smCD3 CD38 or CD8 CD2 CD5 CD34 CD3 (without specifying whether surface membrane staining or cytoplasmic staining should be used for CD3) CD4 CD7, respectively [21]. The later panel was a third generation panel developed based on the previous experience of the same authors with 6-10 color antibody combinations, where the potential utility of markers like CD10 and CD1a+CD34 (both markers conjugated with the same fluorochrome) had been tested and discarded because of their limited additional value for detecting MRD cells in T-ALL/T-LBL [22].

Other conventional flow cytometry panels proposed since 2010 have incorporated a few additional markers for monitoring MRD in T-ALL/T-LBL patients. First, Brammer et al. [23] proposed HLA-DR as a new MRD marker included among a total of 16 antibodies; later on, DiGiuseppe and Wood highlighted the potential utility of CD48 [24]; Patel et al. suggested CD30 and CD79a could be informative MRD markers in T-ALL/T-LBL [25]; and Tembhare et al. included CD117 (conjugated with phycorerythrin-cyanine (PE-Cy) 7) and CD13+CD33 (both conjugated with PE-Cy5.5) [26]. In parallel, other authors have arranged and used their own antibody combinations of between 5 and 15 markers, all of which had been selected from those mentioned in the text above for MRD detection in T-ALL/T-LBL, but they failed to provide the exact composition, in terms of fluorochrome-conjugated antibody clones, used to compose their tubes and panels [27-29].

Despite all efforts made and markers and marker combinations described so far, many pitfalls have not been identified or discussed in the literature, although they hamper the attempts to increase the sensitivity of the flow cytometric MRD detection. According to the EuroFlow experience over the last decade, the main pitfalls and limitations are:
1. Exclusion of cyCD3⁺ NK-cells still remains a major challenge, because CD56 and CD16 are not expressed by all NK-cells, even when both markers are used together (Figure 1A).
2. At the same time, T-ALL/T-LBL cells frequently show positivity for CD56.
3. Downregulation of markers associated with immature T-cell leukemias/lymphomas, such as TdT, CD99, CD34 and CD1a, has been observed in T-ALL/T-LBL patients during or after therapy [22].
4. T-ALL/T-LBL blast cells frequently express high levels of CD45, making them indistinguishable from mature residual T-cells coexisting in the same sample.
5. Importantly, some of the currently used MRD markers, are expressed by T-ALL/T-LBL blasts at lower levels than in mature T-cells, such as CD2, CD4, CD5, CD7 and CD8. These classical T-cell markers are also present in variable percentages of mature T-cells.
6. The same classical T-cell markers are also expressed at low levels in some myeloid cells such as monocytes and dendritic cells (CD2, CD4), myeloid dendritic cells and naive B-cells (CD5), NK-cells (CD2, CD5, CD4, CD7, CD8), CD34⁺ hematopoietic precursors (CD7, CD4) among other hematopoietic cells.
7. Particularly Early T-cell precursor acute lymphoblastic leukemia/lymphoma (ETP-ALL/LBL) and part of the more mature smCD3-positive T-ALL are not easy to separate from NK cells and mature T-lymphocyte subsets.

The present inventors aimed at providing improved reagent compositions that overcome at least part of these limitations and pitfalls and allow for a more optimal discrimination between T-ALL/T-LBL cells and the normal/reactive cells. To that end, they performed extensive testing and searching for marker combinations and improved conjugated antibody reagent panels for more robust identification of T-ALL/T-LBL cells, together with straightforward discrimination from the coexisting normal hematopoietic cells, particularly from normal cyCD3⁺ and cyCD3⁻ NK-cells, CD7⁺ and CD7⁻ CD34⁺ hematopoietic precursors, and mature smCD3⁺ and cyCD3⁺ T-cells.

It was surprisingly found that these goals can be met by staining a sample for the NK-cell associated markers NKp80 and CD16, and the cyCD3 (cytoplasmic CD3) and smCD3 (surface membrane CD3) markers for 1) robust exclusion of all NKp80+ and/or CD16+ NK-cells and cyCD3⁺ smCD3⁺ mature T-cells, and 2) their discrimination from cyCD3⁺ smCD3^{-/lo} T-ALL/T-LBL malignant cells (Table 1A, Figure 1A and 1B).

NKp80, is a type II transmembrane C-type lectin-like receptor. NKp80 is expressed on the cell surface of nearly all human natural killer (NK) cells and a small subset of effector memory CD8+ T cells as a homodimer with approximately 80 kD molecular mass. It stimulates NK cell cytotoxicity and cytokine release and appears to co-operate with other triggering receptors to induce optimal NK cell activation upon interaction with potential target cells. The ligand of NKp80 is AICL (activation-induced C-type lectin), also called CLEC2B. NKp80 is also known as killer cell lectin-like receptor subfamily F, member 1 (KLRF1), or CLEC5C.

Accordingly, the invention provides a reagent composition for the cytometric detection of minimal residual disease (MRD) in T-cell acute lymphoblastic leukemia (T-ALL) and/or T-cell lymphoblastic lymphoma (T-LBL), the reagent composition comprising a panel of at least four antibodies conjugated to a detectable label, the panel comprising at least antibodies against markers NKp80, CD16, cyCD3 and smCD3. Suitably, the antibodies against markers NKp80 and CD16 can be conjugated to distinct labels or to the same detectable label.

The antibody panel may further comprise a conjugated antibody directed against CD6 in order to reach a greater discrimination power between normal CD6+ T-cells and CD6^{-/lo} T-ALL/T-LBL leukemia/lymphoma cells.

The antibody panel can further be combined with CD45RA in another embodiment of this invention, CD45RA being a marker that is aberrantly downregulated in blast cells from most T-ALL/T-LBL patients, compared to naive and effector mature T-cells, thereby allowing for an improved discrimination between T-ALL/T-LBL malignant cells and normal residual T-cells.

In one embodiment, the panel further comprises a set of conjugated antibodies directed against the markers CD117, CD13 and CD33, wherein each antibody within the set is conjugated to a distinct or to the same label.

The reagent composition may further comprise a conjugated antibody directed against CD45.

In one aspect, the panel further comprises conjugated antibodies directed against at least two of the immature T-cell markers CD99, TdT (Terminal deoxynucleotidyl transferase) and CD34, preferably an antibody against CD34 and antibodie(s) against CD99 and/or TdT. For example, the invention provides a reagent composition comprising distinct label-conjugated antibodies directed against the markers CD99, TdT and CD34.

In one embodiment, the panel further comprises distinctly conjugated antibodies against two or more markers selected from the group consisting of CD2, CD5, CD7, CD4 and CD8. Still further, the reagent composition may comprise a conjugated antibody directed against CD38.

According to the invention, the antibodies are suitably conjugated to a fluorochrome. Alternatively, the antibodies are conjugated to a metal isotope, for example an isotope selected from the group consisting of 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt and 209Bi.

The invention also relates to a reagent set comprising two or more distinct reagent compositions herein disclosed. In a specific embodiment, the reagent set comprises or consists of (i) a first reagent composition comprising conjugated antibodies directed against the markers TdT, CD16, NKp80, CD5, cyCD3, CD7, CD45, CD38, CD34, CD45RA, CD6, smCD3, CD117, CD13 and CD33; and (ii) a second reagent composition comprising conjugated antibodies directed against the markers CD99, CD16, NKp80, CD5, cyCD3, CD7, CD45, CD2, CD34, CD45RA, CD8, smCD3 and CD4.

In a specific embodiment, the invention provides a reagent composition or a set of reagents as depicted in any one of Tables 1-7 herein below.

A further aspect relates to a diagnostic kit for cytometric detection of minimal residual disease (MRD) in T-cell acute lymphoblastic leukemia (T-ALL) and/or (T-cell lymphoblastic lymphoma (T-LBL), comprising one or more reagent composition(s) or sets of reagent compositions according to the invention, optionally together with instructions for use, buffer, and/or control samples.

In a further embodiment, the invention provides a cytometric method for detecting minimal residual disease (MRD) in T-cell acute lymphoblastic leukemia (T-ALL) and/or (T-cell lymphoblastic lymphoma (T-LBL), comprising the steps of:
(i) contacting one or more aliquots of a biological sample comprising leukocytes obtained from the subject with a reagent composition according to the invention;
(ii) analyzing leukocytes in said aliquot(s) in a (flow or mass) cytometer; and
(iii) gating on cells for expression of the selected markers detected by the antibodies present in the reagent composition; and
(iv) distinguishing between normal and malignant cells, based on the expression profile of the multiple markers. The method may involve multivariate analysis, preferably principal component analysis (PCA) and/or canonical analysis.

Also provided is the use of a diagnostic kit or method according to the invention, for example in monitoring previously diagnosed T-ALL/T-LBL patients during and/or after therapy. Alternatively, it is advantageously used in patients either suspicious of having T-ALL/T-LBL or after they had been diagnosed with T-ALL/T-LBL, for diagnostic purposes and to assess the extent of tissue infiltration, respectively.

### LEGEND TO THE FIGURES

Figure 1: Illustrating example of the performance of the combined staining with the CD16 and NKp80 (and also CD56 plus NKp80) markers for (A) identification of all human NK-cells, including the NKp80⁻ and CD16⁺ and the NKp80⁺ subsets, as compared to prior art staining with CD16 and CD56 as well as the CD56⁻ and CD56⁺ NK-cell populations; and (B) to discriminate between T-ALL/T-LBL MRD leukemia/lymphoma cells (c) from both cyCD3⁻ (a) and cyCD3⁺ (b) NK-cells in representative bivariate (upper two panels) and multivariate (lower two panels) flow cytometric dot plots from an MRD+ bone marrow of a child with T-ALL/T-LBL (B).

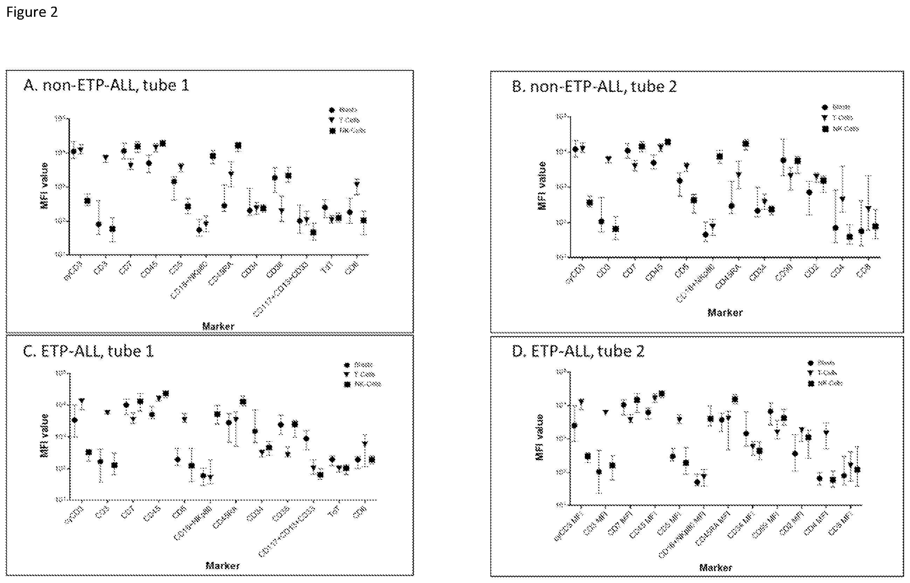

Figure 2: Expression of markers of the 12-color T-MRD panel tubes 1 (A., C.) and 2 (B., D.) on residual blasts (solid circles) of non-ETP-ALL (A.-B.) and ETP-ALL (C.-D.) patients vs. normal T- (solid triangles) and NK-Cells (solid squares) (see Table 6 for marker combinations).

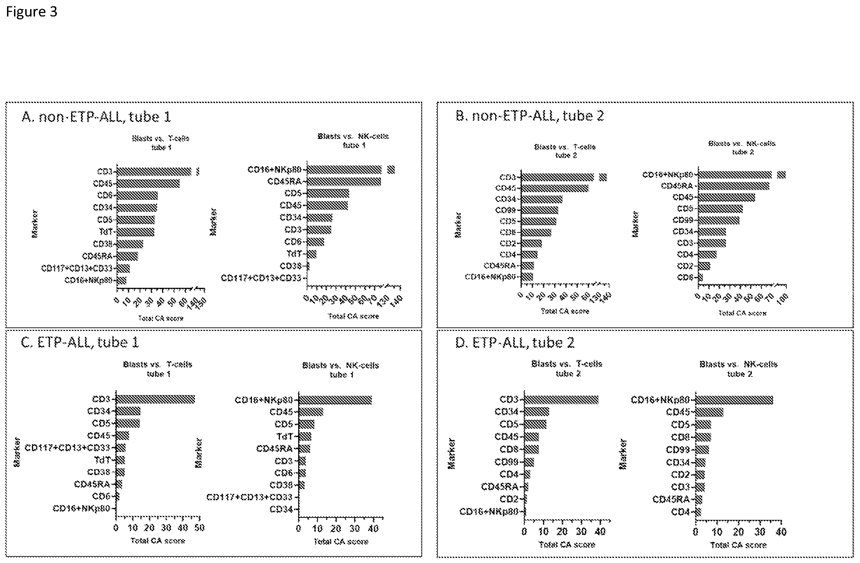

Figure 3: Summary of the contribution of T-MRD panel tube 1 and 2 markers to separation of residual leukemic blasts from normal T- and NK-Cells in MRD-positive non-ETP-ALL (A.-B.; n = 61) and ETP-ALL (C.-D.; n = 16) cases. The ability of markers to discriminate the residual blasts from normal cells was based on a canonical analysis (CA) scoring system performed with the Infinicyt software. Top 4 markers from each respective comparison with the highest contribution to separation received 3, 2, 1 or 0.5 points from the highest to the lowest rank, respectively. So obtained marker scores were summarized independently for comparisons of blasts vs. T-cells and blasts vs. NK-cells in both panel tubes.

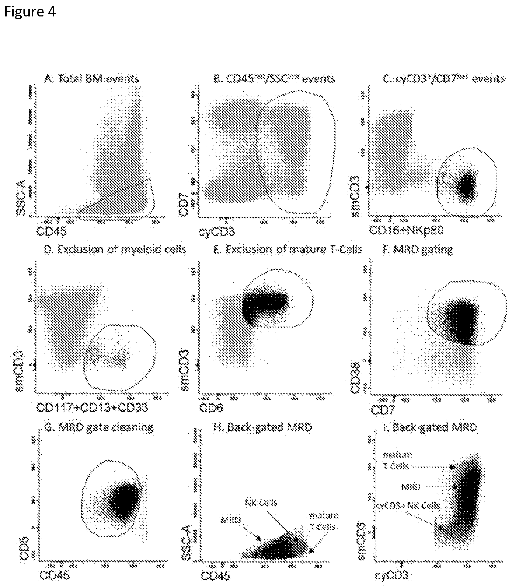

Figure 4: Illustrative example of step-wise gating strategy shown in panels (A) through (G) for identification of MRD in a T-ALL case with partial expression of smCD3. In panel A, events with heterogeneous CD45 and low side scatter (SSC) were gated and shown in panel B, where subsequent gate on events expressing cyCD3, with heterogeneous expression of CD7 was made. In panel C, only the events from the previous step were shown and contaminating CD 16+NKp80-expressing NK cells (black) were gated out. Next, in panel D, myeloid cells (black) expressing CD 117+CD 13+CD33 were gated out and the gate was further cleaned in panel E, where mature smCD3+/CD6+ T-cells (black) were gated out. In subsequent panel F, CD7+/CD38+ MRD population (black) can be distinguished among mature T-Cells and finally separated from mature T-Cells in panel G by gating the CD5low/CD45low population (black). Panels H and I show back-gated MRD population (black), illustrating that the T-ALL blasts cells fall together with mature T-Cells (light grey) and cyCD3+ subset of NK-Cells (dark grey) (indicated with arrows) in classical marker combinations.

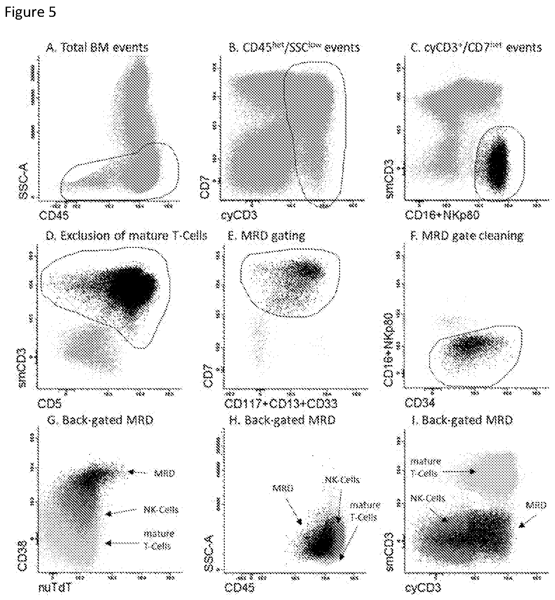

Figure 5: Illustrative example of step-wise gating strategy shown in panels (A) through (F) for identification of MRD in an ETP-ALL case.
In panel A, events with heterogeneous CD45 and low side scatter (SSC) were gated and shown in panel B, where subsequent gate on events expressing cyCD3, with heterogeneous expression of CD7 was made. In panel C, only the events from the previous step were shown and contaminating CD16+NKp80-expressing NK cells (black) were gated out. Next, in panel D, mature smCD3+/CD5+ T-cells (black) were gated out. In panel E, CD7+/CD 117+/CD 13+/CD33+ MRD population (black) can be distinguished, and further cleaned from remaining NK-Cells in panel F. Panels G-I show back-gated MRD population (black) illustrating that the T-ALL blasts cells fall together with mature T-Cells (light grey) and cyCD3+ subset of NK-Cells (dark grey) (indicated with arrows) in classical marker combinations.

### DETAILED DESCRIPTION

The invention relates in some embodiments to improved and advantageous reagent compositions and kits comprising the same. The term "reagent composition" (or "cytometric panel") as used herein relates to a cocktail of antibodies (or other specific antigen-binding agents) which are conjugated to (directly or indirectly) a detectable label.

Antibodies, or immunoglobulins, comprise two heavy chains linked together by disulfide bonds and two light chains, each light chain being linked to a respective heavy chain by disulfide bonds in a "Y" shaped configuration. The variable domains of each pair of light and heavy chains form the antigen binding site. The isotype of the heavy chain (gamma, alpha, delta, epsilon or mu) determines immunoglobulin class (IgG, IgA, IgD, IgE or IgM, respectively). It should be understood that when the terms "antibody" or "antibodies" are used, this is intended to include intact antibodies, such as polyclonal antibodies or monoclonal antibodies (mAbs), as well as proteolytic fragments thereof such as the Fab or F(ab')2 fragments. Further included within the scope of the invention are chimeric antibodies; recombinant and engineered antibodies, and fragments thereof, as well as other molecules comprising at least an antigen binding site (retaining the antigen binding capacity) of an antibody. In a preferred embodiment, the composition comprises a panel of conjugated monoclonal antibodies. (Monoclonal) antibodies against the indicated markers can be commercially obtained from various companies, including Becton/Dickinson (BD) Biosciences, Biolegend, Dako, Beckman Coulter, CYTOGNOS, Caltag, Myltenyi, Pharmingen, Exbio, Sanquin, Invitrogen, and the like.

Antibodies for use in the present invention are conjugated to a label that is detectable by cytometry, e.g. by flow or mass cytometry. Suitable types of detectable labels include fluorochromes (fluorophores), quantum dots, and metal-isotope labels. As will be understood, to facilitate simultaneous detection, all antibodies present in a reagent composition of the invention are conjugated to the same type of detectable label, e.g. all are fluorochrome conjugated or all are isotope labeled. In addition, flow cytometry uses the light properties scattered from cells or particles for identification or quantitative measurement of physical properties.

In one embodiment, the antibody is conjugated to a fluorochrome. A fluorochrome (or "fluorophore") is a chemical which can absorb energy from an excitation source (laser beam) and emit photons at a longer wavelength (fluorescence), which is captured by optical detectors of the flow cytometer. Suitable fluorochromes for conjugating antibodies are known in the art. Each fluorophore has a characteristic peak excitation and emission wavelength, and the emission spectra often overlap. Consequently, the combination of labels which can be used depends on the wavelength of the lamp(s) or laser(s) used to excite the fluorochromes and on the detectors available. The fluorochromes are preferably selected for brightness, limited spectral overlap and limited need for compensation, stability, etc.

A wide range of fluorophores can be used as labels in flow cytometry. Fluorochromes of particular use in a reagent composition according to the invention include those of the Brilliant Violet (BV) series, such as BV421 or functional equivalent thereof, BV510 or functional equivalent thereof, BV605 or functional equivalent thereof, BV650 or functional equivalent thereof, BV711 or functional equivalent thereof, BV786 or functional equivalent thereof; fluorescein isothiocyanate (FITC) or functional equivalent thereof (e.g. BB515) , PerCP Cy5.5 or functional equivalent thereof, phycoerythrin (PE) or functional equivalent thereof, phycoerythrin/CF594 (PE CF594) or functional equivalent thereof, phycoerythrin/cyanine5 (PE-Cy5) or functional equivalent thereof phycoerythrin/cyanine7 (PE-Cy7) or functional equivalent thereof, allophycocyanine (APC) or functional equivalent thereof (Alexa647), AF700 (AlexaFluor700) or functional equivalent thereof, and allophycocyanine/H7 (APC-H7) or functional equivalent thereof.

The following panel of fluorochromes is of particular use in a 12-color reagent composition according to the invention: (L1) fluorescein isothiocyanate (FITC) or functional equivalent thereof (e.g. BB515) (L2) phycoerythrin (PE) or functional equivalent thereof, (L3) PerCP Cy5.5 or functional equivalent thereof (L4) PE-Vio770 or functional equivalent thereof, (L5) allophycocyanine (APC) or functional equivalent thereof (Alexa647), (L6) AlexaFluor700 or functional equivalent thereof, (L7) allophycocyanine/H7 (APC-H7) or functional equivalent thereof, e.g. APC-Cy7, (L8) BV421 or functional equivalent thereof, (L9) BV510 or functional equivalent thereof, (L10) BV605 or functional equivalent thereof; (L11) BV-711 or functional equivalent thereof, (L12) BV-786 or functional equivalent thereof.

In another embodiment, the antibody is conjugated to a quantum dot. Quantum dots are sometimes used in place of traditional fluorophores because of their narrower emission peaks.

In yet another embodiment, the antibody is conjugated to a metal isotope, allowing for detection by mass cytometry, also called cytometry by time-of-flight (CyTOF). Advances in single-cell mass cytometry have increasingly improved highly multidimensional characterization of immune cell heterogeneity. The immunoassay multiplexing capacity relies on monoclonal antibodies labeled with stable heavy-metal isotopes.

Mass cytometry overcomes the fluorescent labeling limit by utilizing lanthanide isotopes attached to antibodies. This method could theoretically allow the use of 40 to 60 distinguishable labels or more. Mass cytometry is fundamentally different from flow cytometry: cells are introduced into a plasma, ionized, and associated isotopes are quantified via time-of-flight mass spectrometry. Although this method permits the use of a large number of labels, it currently has lower throughput capacity than flow cytometry. It also destroys the analysed cells, precluding their recovery by sorting. Finally, a major fraction of cells is lost before reaching the cone for measurement.

To date, a variety of rare-earth elements and noble and post-transition metal isotopes have been used in mass cytometry. For example, Han et al. (2018, Nature Protocols volume 13, pages 2121-2148) disclose protocols for conjugating monoclonal IgG antibodies with 48 high-purity heavy-metal isotopes: (i) 38 isotopes of lanthanides, 2 isotopes of indium, and 1 isotope of yttrium; (ii) 6 isotopes of palladium; and (iii) 1 isotope of bismuth.

In one embodiment, a reagent composition of the invention comprises a panel of antibodies that are conjugated to metals of different mass, in particular isotopes of metals, preferably isotopes of metals selected from the group consisting of Pr, Bi, Y, Nd, Sm, Eu, Gd, Tb, Dy, Ho, Er, Tm, Yb, Lu, Rh,Cd, In, Ir, Pt and Pd. Specific examples include 103Rh, 106Cd, 110Cd, 111Cd, 112Cd, 113Cd, 114Cd, 115In,116Cd, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho,166Er, 167Er, 168Er, 169Tm, 170Er, 171Yb, 172Yb, 173Yb, 174Yb, 175Lu, 176Yb, 1911r, 193Ir, 194Pt, 198Pt and 209Bi.

In a typical process of sample preparation for mass cytometric analysis of cells, cells are incubated (or "stained") with a panel of metal-tagged antibodies that target antigens (markers) of interest. A DNA intercalator can be incorporated into the panel to allow determination of nucleated cells from nonnucleated cells. Cells are stained under resting or stimulating conditions and are fixed prior to analysis. The samples are washed to remove unbound antibody and salts and diluted to an appropriate cell concentration. Cells are then passed in a single-cell suspension into a nebulizer, which aerosolizes the cells into droplets for introduction into the mass cytometer. Upon entering the instrument, cells travel through an argon plasma at 7000°K which completely vaporizes and ionizes the cell and the attached antibodies into a cloud of single-atom ions. The size of the cloud is largely driven by gas expansion kinetics and is relatively independent of the cell size. The ion cloud is filtered by a quadrupole to remove common biological elements with a mass less than ~ 75 Da, to leave only the heavy metal ions that were attached to the staining antibodies directed against the marker set of interest. The ions within the cloud are separated by their mass-to-charge ratio in a time-of-flight (TOF) mass spectrometer. Ion signals are integrated on a per-cell basis, resulting in single-cell measurements for analysis.

The panel of antibodies in a reagent composition provided herein may be used in some embodiments to simultaneously label a cell-containing biological sample (in suspension). In other words, the sample is typically and advantageously incubated with an entire cytometric panel as disclosed herein, thus allowing characterization of multiple cell populations in a single measurement step, using multi-parametric analysis ("multiplexing") of the antigen co-expression pattern on single cells from the sample. Accordingly, antibodies that are directed to distinct cellular targets ("markers") for which separation is desired within a panel, are labeled with distinct (non-equivalent) detectable labels, and are referred to herein as distinct conjugated antibodies. The marker can be a protein that is expressed on a cell's surface (cell surface marker) or in the cytoplasm (cytoplasmic marker; cy marker). Typically, the markers of the present invention are human markers. "CD" stands for cluster designation and is a nomenclature for the identification of specific (human) cell surface antigens defined by monoclonal antibodies.

In some embodiments, a reagent composition comprises antibodies against different markers, but wherein a subset, e.g. two or three, of these antibodies are conjugated to the same detectable label. See for example tube 1 of Table 1A, comprising antibodies against NKp80 and CD16, each conjugated to the same detectable label. The reagent composition may comprise two or more of such pairs of antibodies, 'wherein the antibody within either one of the pairs is conjugated to the same detectable label, but wherein between different pairs the labels are distinguishable. For example, both antibodies of the first pair are conjugated to fluorochrome A and both antibodies of the second pair are conjugated to fluorochrome B. Thus, within each pair the fluorochromes are the same. See for example Table 1D, disclosing reagents comprising antibodies against CD16 and NKp80, each conjugated to label ''L3", and a set of antibodies against CD117, CD13 and CD33 each conjugated to label "L7".

The term "kit" as used herein relates to an article of manufacture comprising at least one and typically a plurality of reagent compositions of the invention, and optionally additional reagents, e.g. reagents for cell dissociation, purification, permeabilization, control samples or antibodies, or other reagents for use in flow cytometry. The kit may further contain instructions for using the at least one reagent composition in the methods of the invention, e.g. instructions for analyzing the results measured using multi-parametric analysis as detailed herein. In one embodiment, the invention provides a 12-color diagnostic kit, comprising one or more 12-color reagent compositions herein disclosed.

A cytometric method for monitoring the immune status and/or the effect of an immune modulatory treatment of a subject typically comprises the steps of: (a) contacting an aliquot of a biological sample comprising leukocytes obtained from the subject with a reagent composition as herein disclosed; (b) analyzing leukocytes in said aliquot in, depending on the type of detectable label, a flow or mass cytometer; and (c) storing and evaluating the data obtained.

For robust, reliable and reproducible flow cytometry-based diagnostics, EuroFlow Consortium has developed new standardized sample preparation methods and standard operating procedures (SOPs). These methods and SOPs allow for high-sensitive detection of MRD by next-generation flow cytometry [7], which are available and described in detail at www.EuroFlow.org.

Any type of sample known or suspected to contain leukocytes may be used directly, or after lysing non-nucleated red cells, or after density centrifugation, or after cell sorting procedures. For example, the sample is peripheral blood, bone marrow, tissue sample such as lymph nodes, adenoid, spleen, or liver, or other type of body fluid such as cerebrospinal fluid, vitreous fluid, synovial fluid, pleural effusions or ascites. Peripheral blood (PB) or bone marrow (BM) is preferred.

Preferably, step (c) of a method provided herein comprises combining the immunophenotypic information of two or more selected cell populations from multiple tubes according to the so-called nearest neighbor calculations in which individual cells from one aliquot of a sample are matched with corresponding individual cells from another aliquot of the same sample, according to their markers and scatter profile. Advantageously, the method comprises the use of software for data integration and multidimensional analysis of flow cytometry files, preferably wherein said software is INFINICYT^{™}.

In the following sections, specific preferred embodiments of the invention are presented. Whereas the embodiments shown relate to antibodies that are conjugated to a fluorochrome as detectable label, it will be understood and appreciated that variant embodiments involving other types of detectable labels, e.g. metal-isotopes, are also encompassed. As used herein, the expression "marker combination" discussed in relation to a reagent composition implies that the composition comprises conjugated antibodies directed at each marker of said marker combination.

For all flow cytometric immunophenotyping MRD studies, fresh samples can be used, such as fresh blood, bone marrow (BM), cerebrospinal fluid, saliva, tears, pleural effusions, peritoneal fluid, bronchoalveolar lavage, thymus, lymph nodes, mediastinal and abdominal masses, and spleen specimens. These samples are suitably stained and processed within 24h after collection. For blood and BM samples, the NH₄Cl-based EuroFlow bulk-lysis standard operating procedure (SOP) may be used to lyse non-nucleated red cells prior to staining (www.EuroFlow.org). Thymus, lymph nodes, mediastinal and abdominal masses, and spleen samples can be mechanically dissociated into single cell suspensions, prior to staining. Thereafter, at least 5x10⁶ leukocytes per sample aliquot may be stained with the appropriate set of monoclonal antibody (Mab) reagents.

For example, in the stainings performed with the 12-color 2-tube T-ALL/T-LBL reagent set as herein disclosed, the following 19 Mab reagents were used: smCD3 (UCHT1) - Brilliant Violet-711 (BV-711) (Becton Dickinson, BD); cyCD3 (REA613) - phycoerythrin-Vio-770 (PE-Vio-770) (Miltenyi); CD5 (L17F12) - peridinin-chlorophyll protein-Cy5.5 (PerCP-Cy5.5) (BD); CD7 (124-1D1) - allophycocyanin (APC) (Thermo Fisher); CD45 (2D1) - Alexa Fluor-700 (AF-700) (Biolegend); CD45RA (HI100) - Brilliant Violet-510 (BV-510) (BD); CD6 (M-T605) - Brilliant Violet-605 (BV-605) (BD); CD34 (8G12) - Brilliant Violet-421 (BV-421) (BD); CD99 (3B2/TA8) - fluorescein isothiocyanate (FITC) (Biolegend); CD16 (B73.1) - phycoerythrin (PE) (BD); NKp80 (4A4.D10) - phycoerythrin (PE) (Miltenyi); terminal nucleotide transferase (TdT) (HT-6) - fluorescein isothiocyanate (FITC) (Agilent); CD117 (104D2) - Brilliant Violet-786 (BV-786) (BD); CD13 (L138) - Brilliant Violet-786 (BV-786) (BD); CD33 (P67.6) - Brilliant Violet-786 (BV-786) (BD); CD4 (SK3) - Brilliant Violet-786 (BV-786) (BD); CD8 (SK1) - Brilliant Violet-605 (BV-605) (BD); CD38 (HB7) - allophycocyanin-hilite7 (APC-H7) (BD); CD2 (RPA-2.10) - allophycocyanin-hilite7 (APC-H7) (BD).

Acquisition of the stained samples is preferably performed immediately after immunostaining, in a FACS Lyric flow cytometer (BD), using the FACSDiva^{™} software (BD). For instrument setup, calibration and monitoring, the EuroFlow instrument setup & compensation protocol for 14-color measurements may be used [30,31]. For data analysis, the Infinicyt^{™} software (Cytognos S.L.) can be employed. For each blood sample, relative and absolute (double-platform) cell counts are calculated and recorded for both the major cell population of interest and each specific cell subset. In BM, lymph node, thymus and spleen samples, only relative cell numbers may be derived.

In a first embodiment of the invention, a sample is stained with a reagent composition comprising an antibody panel directed against the NK-cell associated markers NKp80 and CD16, and the cyCD3 and smCD3 markers. This novel panel allows for 1) the robust exclusion of all NKp80⁺ and/or CD16⁺ NK-cells and cyCD3⁺ smCD3⁺ mature T-cells, and 2) their discrimination from cyCD3⁺ smCD3^{-/lo} T-ALL/T-LBL malignant cells (Table 1A, Figure 1A and 1B).

In a preferred embodiment, the antibodies against the NKp80 and CD16 markers are conjugated to the same detectable label (e.g. fluorochrome or metal isotope) while the antibodies against cyCD3 and smCD3 are each conjugated to different (second and third) labels, respectively (Table 1A, Figure 1A and 1B). In a preferred embodiment, the antibody reagents against the NKp80 and CD16 markers can be both combined with the same label while cyCD3 and smCD3 are conjugated each with different (second and third) labels, respectively (Table 1A, Figure 1A and 1B).

Alternatively, antibodies directed against NKp80 and CD16 are each conjugated to a different label (Table 1A). Then, ≥ 10⁷ cellular events of the stained sample can be measured in a flow cytometer, and flow cytometry data may be analyzed using conventional manual gating or automated gating procedures, based on commercially available flow cytometry data analysis software programs.

For a broader applicability and greater sensitivity, the NKp80, CD16, cyCD3 and smCD3 markers are advantageously combined with CD45RA. CD45RA is a marker that is aberrantly downregulated in blast cells from most T-ALL/T-LBL patients compared to naive and terminal effector mature T-cells (Table 1B), thereby allowing for an improved discrimination between T-ALL/T-LBL malignant cells and normal residual T-cells.

Alternatively or additionally, an antibody against CD6 is added to the antibody panel for the NKp80, CD16, cyCD3 and smCD3 marker combination or the NKp80, CD16, cyCD3, smCD3 and CD45RA marker combination. This allows to reach a greater discrimination power between normal CD6⁺ T-cells and CD6^{-/lo} T-ALL/T-LBL leukemia/lymphoma cells (Table 1C).

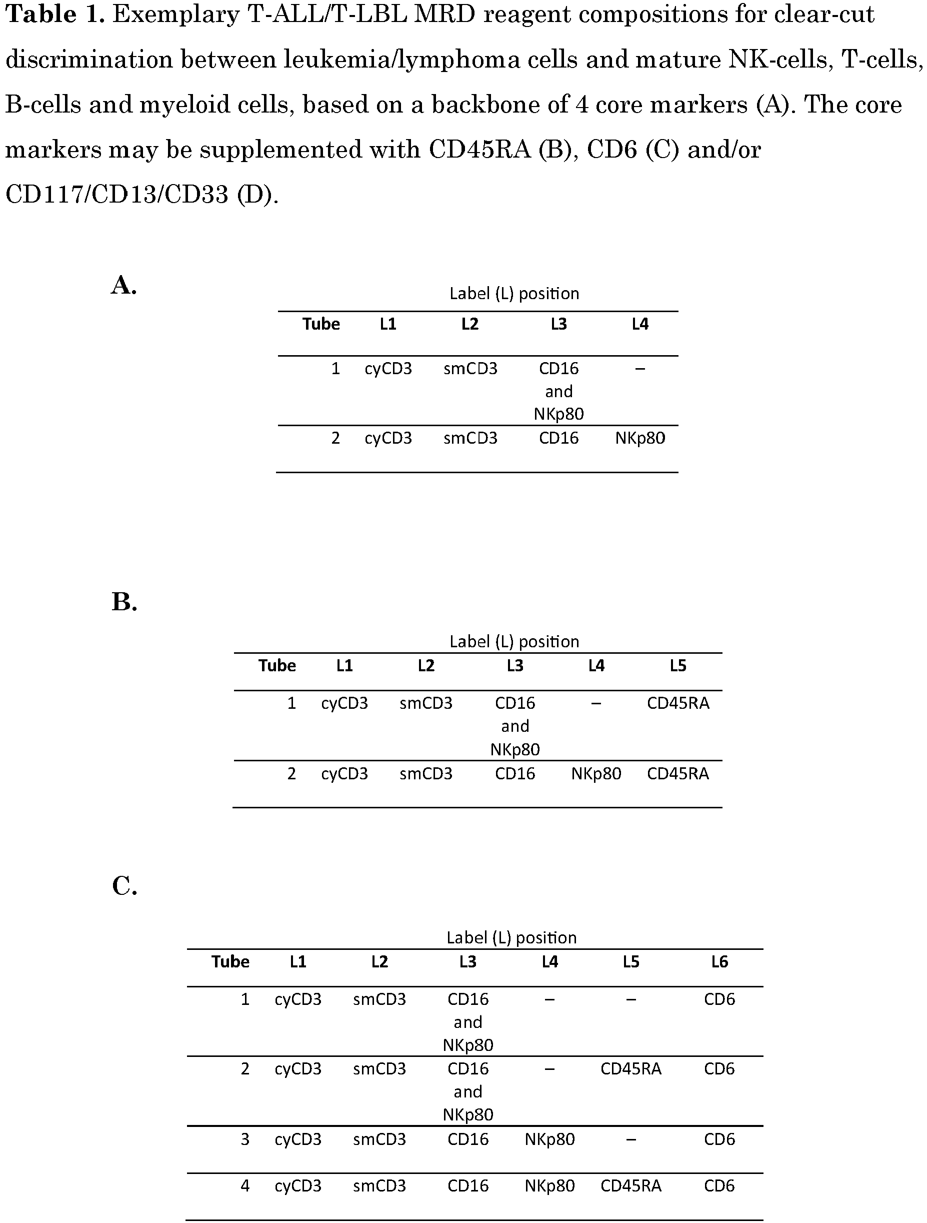

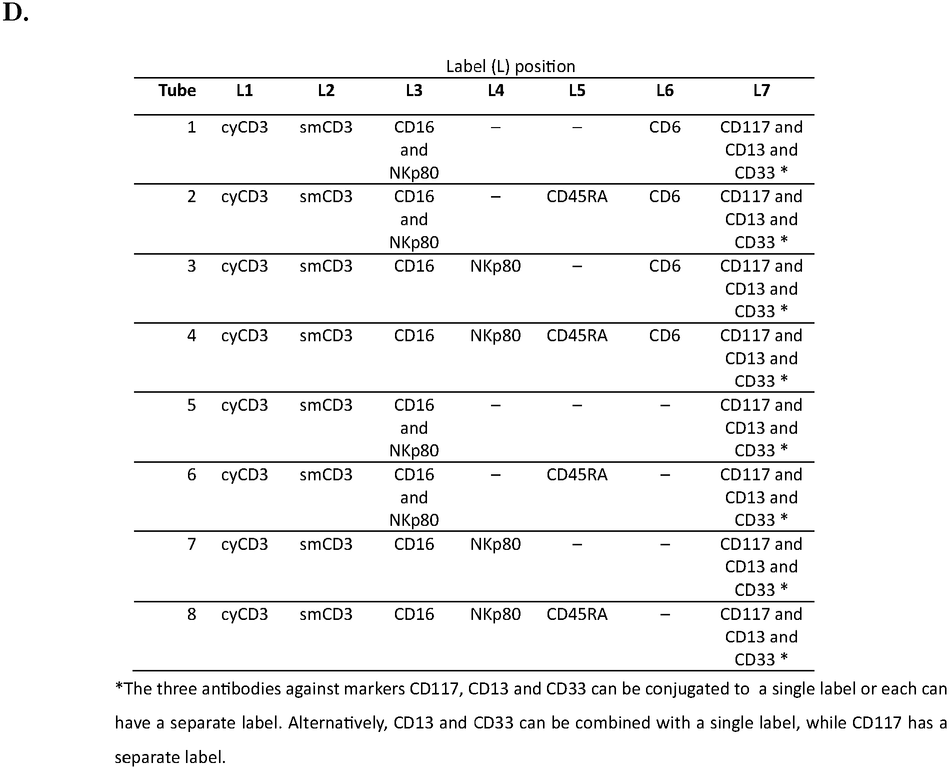

Still further, antibodies directed against the CD 117+CD 13+CD33 myeloid and stem cell-associated markers, conjugated all three to the same detectable label, may also be added to the antibody panel against each of the above marker combinations. This allows for a higher applicability and for more sensitive discrimination between T-ALL/T-LBL malignant cells and normal hematopoietic lymphoid cells (Table 1D).

In yet another embodiment of this invention, an antibody against the CD45 marker conjugated to a different, unique detectable label is added to any of the above said antibody combinations, for more accurate identification and enumeration of T-ALL/T-LBL malignant cells (Table 2).

**Table 2. Exemplary reagent compositions for clear-cut discrimination between leukemia/lymphoma cells and mature NK-cells, T-cells, B-cells and myeloid cells, and identification of different populations of leukocytes by including an antibody against CD45.**

| Label (L) position | | | | | | | | |
|---|---|---|---|---|---|---|---|---|
| **Tube** | **L1** | **L2** | **L3** | **L4** | **L5** | **L6** | **L7** | **L8** |
| 1 | cyCD3 | smCD3 | CD16 and NKp80 | - | - | - | - | CD45 |
| 2 | cyCD3 | smCD3 | CD16 | NKp80 | - | - | - | CD45 |
| 3 | cyCD3 | smCD3 | CD16 and NKp80 | - | CD45RA | - | - | CD45 |
| 4 | cyCD3 | smCD3 | CD16 | NKp80 | CD45RA | - | - | CD45 |
| 5 | cyCD3 | smCD3 | CD16 and NKp80 | - | - | CD6 | - | CD45 |
| 6 | cyCD3 | smCD3 | CD16 and NKp80 | - | CD45RA | CD6 | - | CD45 |
| 7 | cyCD3 | smCD3 | CD16 | NKp80 | - | CD6 | - | CD45 |
| 8 | cyCD3 | smCD3 | CD16 | NKp80 | CD45RA | CD6 | - | CD45 |
| 9 | cyCD3 | smCD3 | CD16 and NKp80 | - | - | CD6 | CD117 and CD13 and CD33 | CD45 |
| 10 | cyCD3 | smCD3 | CD16 and NKp80 | - | CD45RA | CD6 | CD117 and CD13 and CD33 | CD45 |
| 11 | cyCD3 | smCD3 | CD16 | NKp80 | - | CD6 | CD117 and CD13 and CD33 | CD45 |
| 12 | cyCD3 | smCD3 | CD16 | NKp80 | CD45RA | CD6 | CD117 and CD13 and CD33 | CD45 |
| 13 | cyCD3 | smCD3 | CD16 and NKp80 | - | - | - | CD117 and CD13 and CD33 | CD45 |
| 14 | cyCD3 | smCD3 | CD16 and NKp80 | - | CD45RA | - | CD117 and CD13 and CD33 | CD45 |
| 15 | cyCD3 | smCD3 | CD16 | NKp80 | - | - | CD117 and CD13 and CD33 | CD45 |
| 16 | cyCD3 | smCD3 | CD16 | NKp80 | CD45RA | - | CD117 and CD13 and CD33 | CD45 |

Suitably, the CD99, TdT and CD34 immature T-cell markers are added to each of the above antibody combinations (Table 3A). Alternatively, if required for limited usage of labels, two of the above three immature markers, preferably CD34 and CD99 (Table 3B) or CD34 and TdT (Table 3C) are selected for inclusion in any of the above antibody combinations.

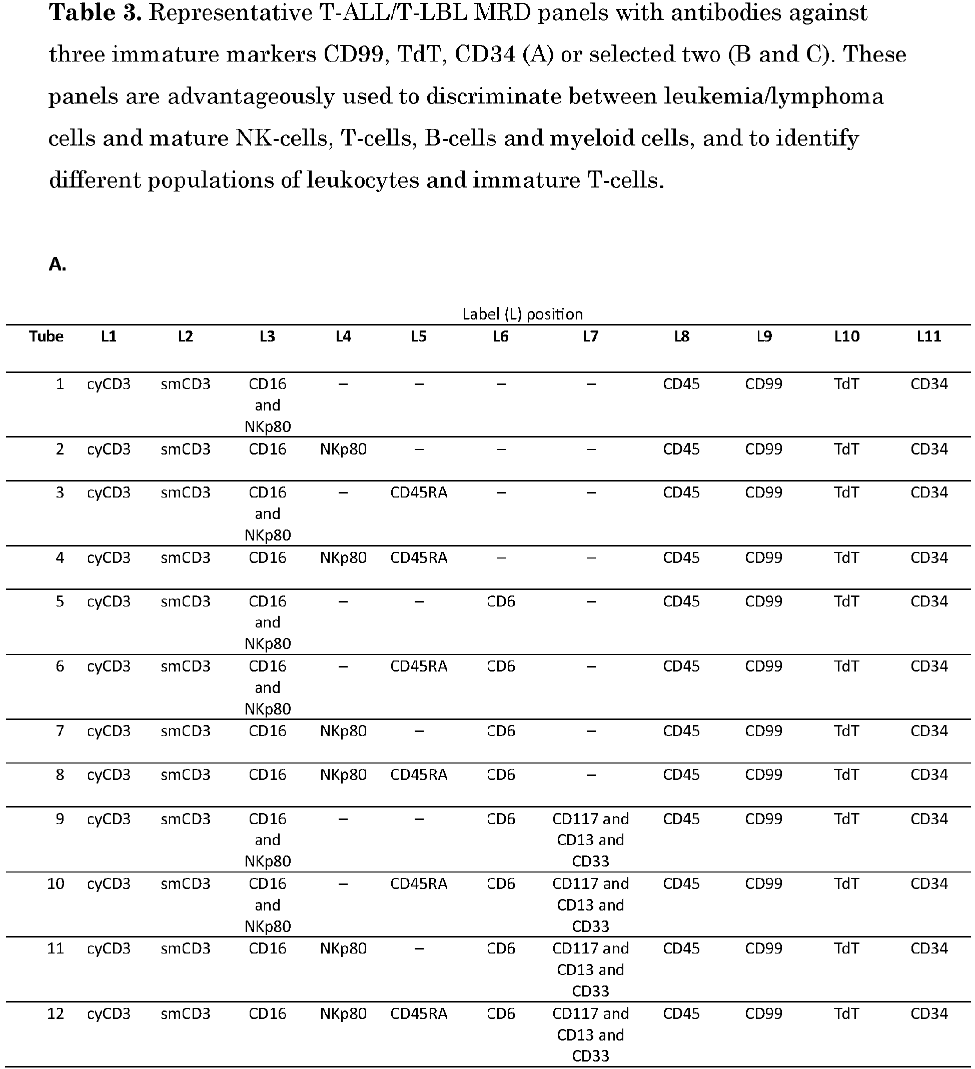

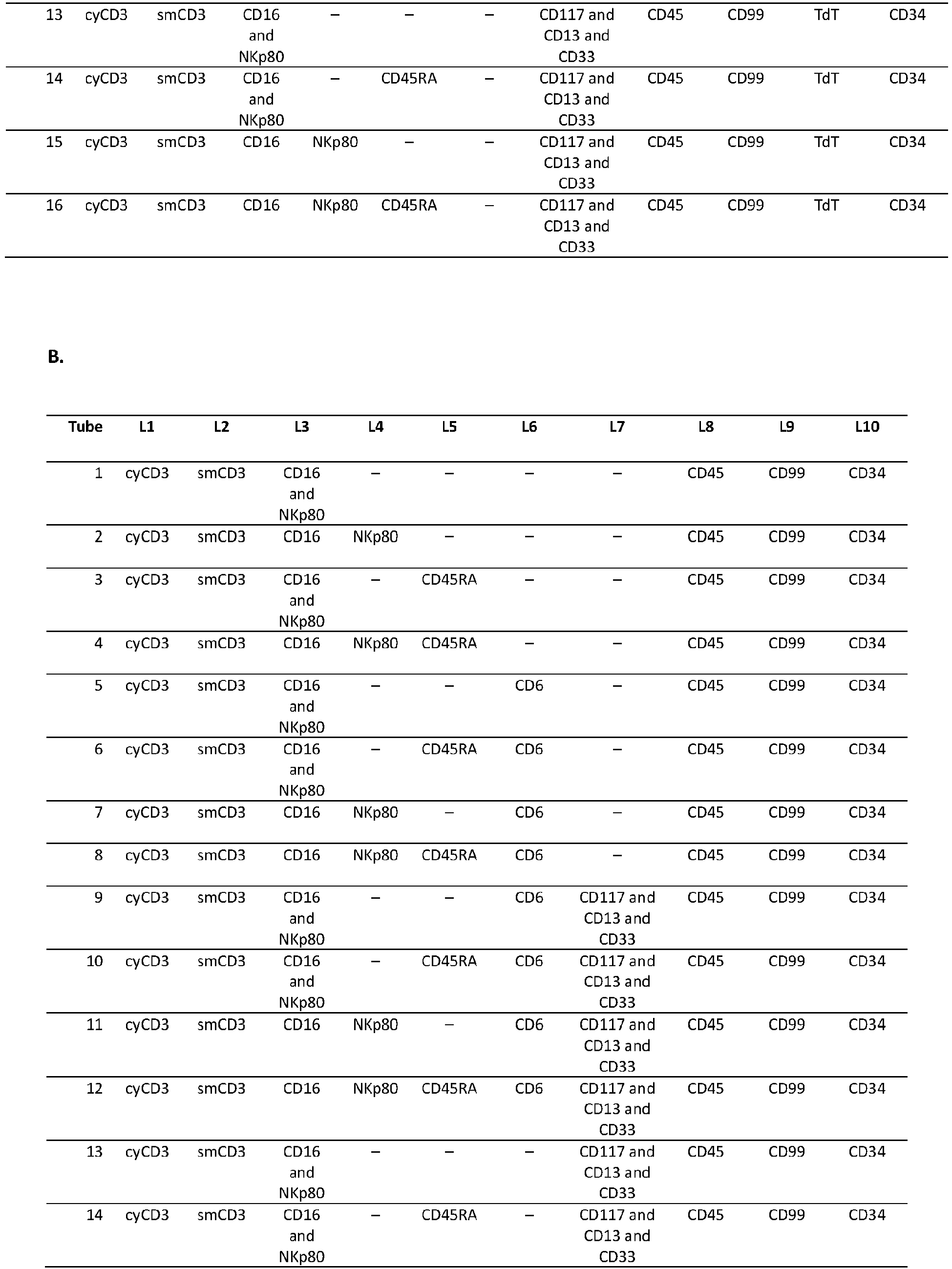

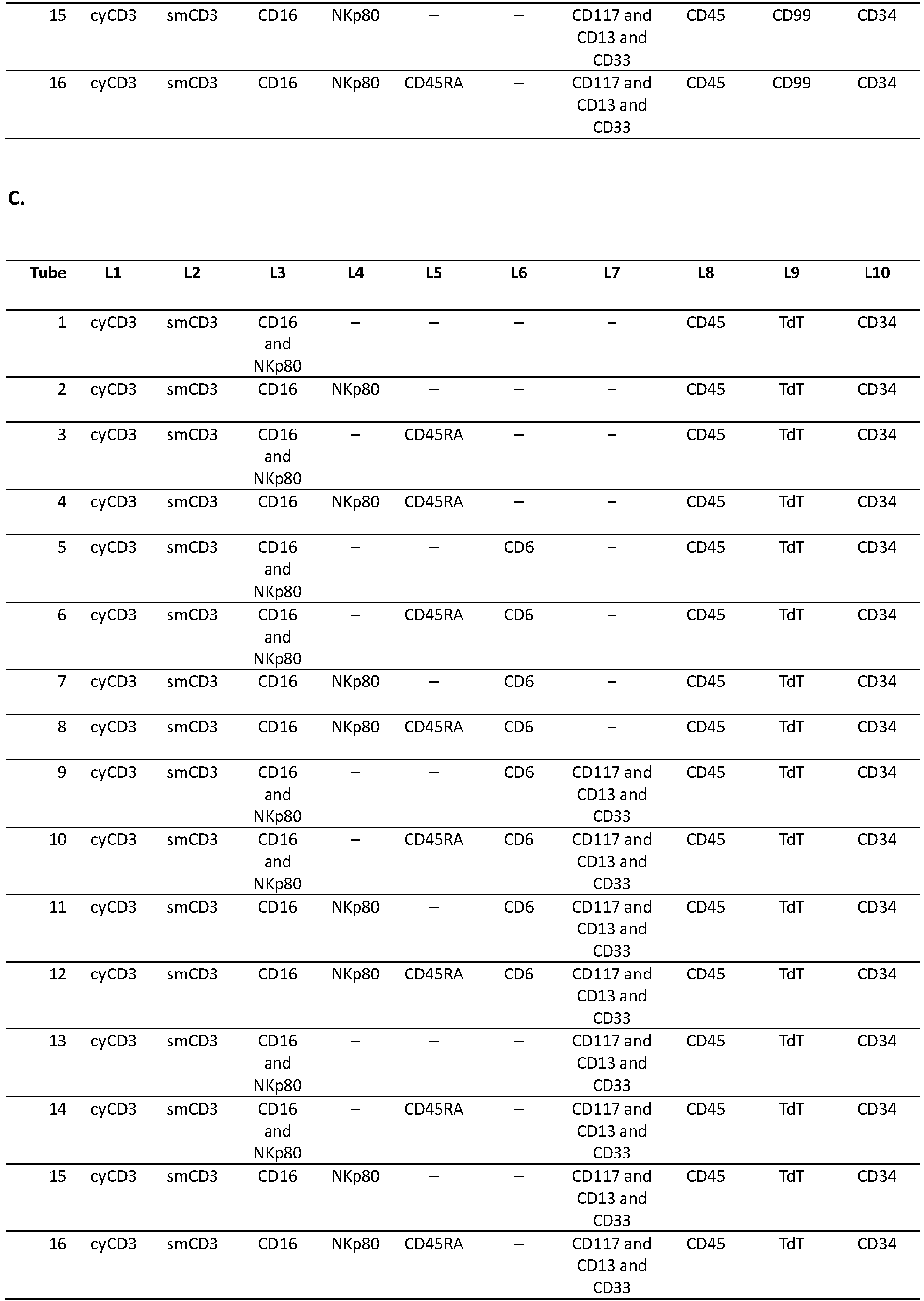

In a further embodiment of this invention, antibodies against two or more markers selected from the mature T-cell associated markers CD2, CD5, CD7, CD4 and CD8 are combined with any one of the above listed antibody mixtures to provide an admixture of antibodies conjugated with up to between 15 (cyCD3, smCD3, CD16+NKp80, CD45RA, CD6, CD117+CD33+CD13, CD45, CD99, TdT, CD34, CD2, CD5, CD7, CD4, CD8) and 18 (cyCD3, smCD3, CD16, NKp80, CD45RA, CD6, CD117, CD33, CD13, CD45, CD99, TdT, CD34, CD2, CD5, CD7, CD4, CD8) different labels, as illustrated in Table 4.

In another embodiment, the CD38 marker is added to the above listed 15 and 18 antibody combinations into 16-antibody and 19-antibody mixtures (Table 5), for improved identification of T-ALL/T-LBL malignant cells and to assess the levels of expression of the CD38 protein on said T-ALL/T-LBL malignant cells, as a potential target protein on tumor cells for selection of next therapy.

**Table 4. Exemplary antibody panels for clear-cut discrimination between leukemia/lymphoma cells and mature NK-cells, T-cells, B-cells and myeloid cells, and identification of different populations of leukocytes and immature T-cells and detailed subsets of mature T-cells with the use of CD2, CD5, CD7, CD4 and CD8 markers.**

| Label (L) position | | | | | | | | | | | | | | | | |
|---|---|---|---|---|---|---|---|---|---|---|---|---|---|---|---|---|
| Tube | **L1** | **L2** | **L3** | **L4** | **L5** | **L6** | **L7** | **L8** | **L9** | **L10** | **L11** | **L12** | **L13** | **L14** | **L15** | **L16** |
| 1 | cyCD3 | smCD3 | CD16 and NKp80 | - | - | - | - | CD45 | TdT | CD99 | CD34 | CD2 | CD5 | CD7 | CD4 | CD8 |
| 2 | cyCD3 | smCD3 | CD16 | NKp80 | - | - | - | CD45 | TdT | CD99 | CD34 | CD2 | CD5 | CD7 | CD4 | CD8 |
| 3 | cyCD3 | smCD3 | CD16 and NKp80 | - | CD45RA | - | - | CD45 | TdT | CD99 | CD34 | CD2 | CD5 | CD7 | CD4 | CD8 |
| 4 | cyCD3 | smCD3 | CD16 | NKp80 | CD45RA | - | - | CD45 | TdT | CD99 | CD34 | CD2 | CD5 | CD7 | CD4 | CD8 |
| 5 | cyCD3 | smCD3 | CD16 and NKp80 | - | - | CD6 | - | CD45 | TdT | CD99 | CD34 | CD2 | CD5 | CD7 | CD4 | CD8 |
| 6 | cyCD3 | smCD3 | CD16 and NKp80 | - | CD45RA | CD6 | - | CD45 | TdT | CD99 | CD34 | CD2 | CD5 | CD7 | CD4 | CD8 |
| 7 | cyCD3 | smCD3 | CD16 | NKp80 | - | CD6 | - | CD45 | TdT | CD99 | CD34 | CD2 | CD5 | CD7 | CD4 | CD8 |
| 8 | cyCD3 | smCD3 | CD16 | NKp80 | CD45RA | CD6 | - | CD45 | TdT | CD99 | CD34 | CD2 | CD5 | CD7 | CD4 | CD8 |
| 9 | cyCD3 | smCD3 | CD16 and NKp80 | - | - | CD6 | CD117 and CD13 and CD33 | CD45 | TdT | CD99 | CD34 | CD2 | CD5 | CD7 | CD4 | CD8 |
| 10 | cyCD3 | smCD3 | CD16 and NKp80 | - | CD45RA | CD6 | CD117 and CD13 and CD33 | CD45 | TdT | CD99 | CD34 | CD2 | CD5 | CD7 | CD4 | CD8 |
| 11 | cyCD3 | smCD3 | CD16 | NKp80 | - | CD6 | CD117 and CD13 and CD33 | CD45 | TdT | CD99 | CD34 | CD2 | CD5 | CD7 | CD4 | CD8 |
| 12 | cyCD3 | smCD3 | CD16 | NKp80 | CD45RA | CD6 | CD117 and CD13 and CD33 | CD45 | TdT | CD99 | CD34 | CD2 | CD5 | CD7 | CD4 | CD8 |
| 13 | cyCD3 | smCD3 | CD16 and NKp80 | - | - | - | CD117 and CD13 and CD33 | CD45 | TdT | CD99 | CD34 | CD2 | CD5 | CD7 | CD4 | CD8 |
| 14 | cyCD3 | smCD3 | CD16 and NKp80 | - | CD45RA | - | CD117 and CD13 and CD33 | CD45 | TdT | CD99 | CD34 | CD2 | CD5 | CD7 | CD4 | CD8 |
| 15 | cyCD3 | smCD3 | CD16 | NKp80 | - | - | CD117 and CD13 and CD33 | CD45 | TdT | CD99 | CD34 | CD2 | CD5 | CD7 | CD4 | CD8 |
| 16 | cyCD3 | smCD3 | CD16 | NKp80 | CD45RA | - | CD117 and CD13 and CD33 | CD45 | TdT | CD99 | CD34 | CD2 | CD5 | CD7 | CD4 | CD8 |

**Table 5. Exemplary T-ALL/T-LBL MRD panels for clear-cut discrimination between leukemia/lymphoma cells and mature NK-cells, T-cells, B-cells and myeloid cells, and identification of different populations of leukocytes and immature T-cells and detailed subsets of mature T-cells supplemented with aberrancy marker of CD38.**

| Label (L) position | | | | | | | | | | | | | | | | | |
|---|---|---|---|---|---|---|---|---|---|---|---|---|---|---|---|---|---|
| Tube | **L1** | **L2** | **L3** | **L4** | **L5** | **L6** | **L7** | **L8** | **L9** | **L10** | **L11** | **L12** | **L13** | **L14** | **L15** | **L16** | **L17** |
| 1 | cyCD3 | smCD3 | CD16 and NKp80 | - | - | - | - | CD45 | TdT | CD99 | CD34 | CD2 | CD5 | CD7 | CD4 | CD8 | CD38 |
| 2 | cyCD3 | smCD3 | CD16 | NKp80 | - | - | - | CD45 | TdT | CD99 | CD34 | CD2 | CD5 | CD7 | CD4 | CD8 | CD38 |
| 3 | cyCD3 | smCD3 | CD16 and NKp80 | - | CD45RA | - | - | CD45 | TdT | CD99 | CD34 | CD2 | CD5 | CD7 | CD4 | CD8 | CD38 |
| 4 | cyCD3 | smCD3 | CD16 | NKp80 | CD45RA | - | - | CD45 | TdT | CD99 | CD34 | CD2 | CD5 | CD7 | CD4 | CD8 | CD38 |
| 5 | cyCD3 | smCD3 | CD16 and NKp80 | - | - | CD6 | - | CD45 | TdT | CD99 | CD34 | CD2 | CD5 | CD7 | CD4 | CD8 | CD38 |
| 6 | cyCD3 | smCD3 | CD16 and NKp80 | - | CD45RA | CD6 | - | CD45 | TdT | CD99 | CD34 | CD2 | CD5 | CD7 | CD4 | CD8 | CD38 |
| 7 | cyCD3 | smCD3 | CD16 | NKp80 | - | CD6 | - | CD45 | TdT | CD99 | CD34 | CD2 | CD5 | CD7 | CD4 | CD8 | CD38 |
| 8 | cyCD3 | smCD3 | CD16 | NKp80 | CD45RA | CD6 | - | CD45 | TdT | CD99 | CD34 | CD2 | CD5 | CD7 | CD4 | CD8 | CD38 |
| 9 | cyCD3 | smCD3 | CD16 and NKp80 | - | - | CD6 | CD117 and CD13 and CD33 | CD45 | TdT | CD99 | CD34 | CD2 | CD5 | CD7 | CD4 | CD8 | CD38 |
| 10 | cyCD3 | smCD3 | CD16 and NKp80 | - | CD45RA | CD6 | CD117 and CD13 and CD33 | CD45 | TdT | CD99 | CD34 | CD2 | CD5 | CD7 | CD4 | CD8 | CD38 |
| 11 | cyCD3 | smCD3 | CD16 | NKp80 | - | CD6 | CD117 and CD13 and CD33 | CD45 | TdT | CD99 | CD34 | CD2 | CD5 | CD7 | CD4 | CD8 | CD38 |
| 12 | cyCD3 | smCD3 | CD16 | NKp80 | CD45RA | CD6 | CD117 and CD13 and CD33 | CD45 | TdT | CD99 | CD34 | CD2 | CD5 | CD7 | CD4 | CD8 | CD38 |
| 13 | cyCD3 | smCD3 | CD16 and NKp80 | - | - | - | CD117 and CD13 and CD33 | CD45 | TdT | CD99 | CD34 | CD2 | CD5 | CD7 | CD4 | CD8 | CD38 |
| 14 | cyCD3 | smCD3 | CD16 and NKp80 | - | CD45RA | - | CD117 and CD13 and CD33 | CD45 | TdT | CD99 | CD34 | CD2 | CD5 | CD7 | CD4 | CD8 | CD38 |
| 15 | cyCD3 | smCD3 | CD16 | NKp80 | - | - | CD117 and CD13 and CD33 | CD45 | TdT | CD99 | CD34 | CD2 | CD5 | CD7 | CD4 | CD8 | CD38 |
| 16 | cyCD3 | smCD3 | CD16 | NKp80 | CD45RA | - | CD117 and CD13 and CD33 | CD45 | TdT | CD99 | CD34 | CD2 | CD5 | CD7 | CD4 | CD8 | CD38 |

**Table 6. Marker composition of 2-tube, 12-color T-ALL/T-LBL MRD panel of the invention for flow and spectral cytometry (A) with examples of possible labels (fluorochromes) (B).**

| | **A.** | | | | | | | | | | | |
|---|---|---|---|---|---|---|---|---|---|---|---|---|
| Label (L) position | | | | | | | | | | | | |
| **Tube** | **L1** | **L2** | **L3** | **L4** | **L5** | **L6** | **L7** | **L8** | **L9** | **L10** | **L11** | **L12** |
| 1 | TdT | CD16 and NKp80 | CD5 | cyCD3 | CD7 | CD45 | CD38 | CD34 | CD45RA | CD6 | CD3 | CD117 and CD13 and CD33 |
| 2 | CD99 | CD16 and NKp80 | CD5 | cyCD3 | CD7 | CD45 | CD2 | CD34 | CD45RA | CD8 | CD3 | CD4 |

| | **B.** | | | | | | | | | | | |
|---|---|---|---|---|---|---|---|---|---|---|---|---|
| **Tube** | **FITC** | **PE** | **PerCP -Cy5.5** | **PE-Vio770** | **APC** | **Alexa Fluor 700** | **APC-H7** | **BV-421** | **BV-510** | **BV-605** | **BV-711** | **BV-786** |
| 1 | TdT | CD16 and NKp80 | CD5 | cyCD3 | CD7 | CD45 | CD38 | CD34 | CD45RA | CD6 | CD3 | CD117 and CD13 and CD33 |
| 2 | CD99 | CD16 and NKp80 | CD5 | cyCD3 | CD7 | CD45 | CD2 | CD34 | CD45RA | CD8 | CD3 | CD4 |

**Table 7. Exemplary 19-parameter T-ALL/T-LBL MRD reagent composition for detection by mass cytometry.**

| **F1** | **F2** | **F3** | **F4** | **F5** | **F6** | **F7** | **F8** | **F9** | **F10** | **F11** | **F12** | **F13** | **F14** | **F15** | **F16** | **F17** | **F18** | **F19** |
|---|---|---|---|---|---|---|---|---|---|---|---|---|---|---|---|---|---|---|
| TdT | CD16 | NKp80 | CD5 | cyCD3 | CD7 | CD45 | CD38 | CD34 | CD45RA | CD6 | CD3 | CD117 | CD13 | CD33 | CD99 | CD2 | CD8 | CD4 |

In some embodiments, the above listed 16-color and 19-color antibody mixtures can be divided in two 12-color reagent compositions. Preferably, antibodies against the marker panel CD16+NKp80, cyCD3, smCD3, CD45RA, CD45, CD34 and CD7 are combined with TdT, CD38, CD6 and CD117+CD33+CD13 in a first tube, and the former marker panel is combined with antibodies against the CD99, CD2, CD4 and CD8 markers in a second tube (Table 6A). Table 6B provides an exemplary set of fluorochromes to be used in the above described 12-color setting.

Taking into account the above-described innovative steps, a new approach is proposed which allows for high-sensitive MRD detection in T-ALL/T-LBL at levels below 10⁻⁵, down to 10⁻⁶ whenever sufficient cells are evaluated.

In a preferred embodiment, the invention is used in previously diagnosed T-ALL/T-LBL patients either during or after therapy. Alternatively, it is also used in patients either suspicious of having T-ALL/T-LBL or after they had been diagnosed with T-ALL/T-LBL, for diagnostic purposes and to assess the extent of tissue infiltration, respectively.

In one embodiment, an antibody panel herein disclosed is used for detecting MRD in patients with Early T-cell precursor (ETP) acute lymphoblastic leukemia/lymphoma (ALL/LBL). This is a newly recognized entity of T-lymphoblastic leukemia/lymphoma. ETP-ALL occurs more frequently than initially assumed (5 to 10% in childhood T-ALL patients and 10 to 15 % or more in adult T-ALL patients) and concerns a particular challenging T-ALL subgroup with respect to MRD monitoring. ETP-ALL has distinct characteristics compared with other subtypes of T-ALL, and has attracted attention given its refractoriness to chemotherapy. The origin of ETP-ALL is considered to involve the migration of cells from the thymus to the bone marrow (BM). These cells, although they have characteristics of T-cell lineage commitment, continue to have the potential for myeloid/dendritic cell differentiation. See Puglianini et al. (Ther Adv Hematol., 2020, Vol. 11: 1-13) reviewing the features of ETP-ALL, diagnostic challenges, and treatment outcomes.

The described invention is aimed to be used in bone marrow, blood and other body tissues and body fluids, such as cerebrospinal fluid, saliva, tears, pleural effusions, peritoneal fluid, bronchoalveolar lavage, lymph nodes, thymus, mediastinal and abdominal masses. Blood containing samples are collected in K₂EDTA, K₃EDTA, heparin or citrate as anticoagulant. Alternatively, they can be stained fresh or after stabilization with commercially available reagents like Transfix^{™}.

In one embodiment of this invention, staining of samples is performed in the whole sample, after erythrocyte lysis or on mononuclear cells isolated from the whole sample using conventional techniques for staining of cell surface membrane markers plus cytoplasmic markers, including, but not limited to the EuroFlow standard operating procedures for bulk lysis and for staining of cell surface membrane and cytoplasmic markers, which detailed description is available at www.EuroFlow.org.

In one embodiment of this invention, antibody reagents used to stain the above listed types of sample are conjugated with labels selected from (but not limited to) the following list of compatible fluorochromes: BUV395, DyLight 350, BUV496, BUV563, BUV615, BUV661, BUV737, BUV805, BV421, Pacific Blue, BV480, eFluor506, BV510, BV570, BV605, BV650, BV711, BV750, BV786, BB515, FITC, CF514, PerCP, BB700, PerCP-Cy5.5, PerCP-eFluor710, BB790-P, PE, CF568, PE-Dazzle594, CF594, PE-AF610, PE-Cy5, PE-Cy5.5, PE-Cy7, APC, AF647, Spark NIR 685, APC-R700, AF700, APC-Vio770, APC-Fire810, sparkBlue550, realBlue780, realyellow586, PE-Vio615, PE-Fire640, PE-Fire700, PE-Fire810, APC-C750, R718, APC705.

In one embodiment, antibody reagents are conjugated with labels selected from (but not limited to) the following list of compatible isotopes: 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt, 209Bi.

As will be appreciated by a person skilled in the art, detection of T-ALL/T-LBL leukemia/lymphoma cells according to the invention can be readily performed using conventional flow cytometry, next generation flow cytometry, spectral cytometry or mass cytometry (Table 7).

### EXPERIMENTAL SECTION

The invention is exemplified by the example below, which is provided for illustrating purposes and in no manner limits the scope.

### EXAMPLE 1: Analysis of bone marrow and peripheral blood samples

*Sample collection.* For MFC analyses, total of 126 follow-up bone marrow (BM, n=109) and peripheral blood (PB, n=17) samples were collected at EDTA as anticoagulant at different time points of the therapy (Induction n=71, post induction n=13, consolidation n=16, maintenance n=11, end of therapy n=4, post- hematopoietic stem cell transplantation n=6, during relapse n=5) with possible multiple samples collected from the same patient. All BM and PB samples were processed locally with standardized sample staining protocols, including bulk lysis protocol for staining ≥5x10⁶ cells in follow-up T-ALL/T-LBL samples, as well as with standardized cytometer set-up, calibration and staining procedures developed by the EuroFlow Consortium described previously and available at https://euroflow.org/protocols (Kalina et al. 2012; Van Dongen et al. 2012). Briefly, 2ml of each sample plus 50 mL of ammonium chloride lysing solution were mixed in a 50ml Falcon tube and incubated for 15 min in a sample-shaker device. Afterward, the sample was centrifuged at 800g for 10 min and the supernatant discarded using a Pasteur pipette without disturbing the cell pellet. Upon discarding the supernatant, the tube was refilled with PBS containing 0.09% NaN₃ and 0.5 % BSA to a final volume of 50 ml and centrifuged once again at 800g (5 min). Without disturbing the cell pellet, the supernatant was discarded, and the cell pellet was resuspended in 2 mL of PBS with 0.09% NaN₃ and 0.5 % BSA. Then, the cells were transferred to a 5 mL polystyrene roundbottom Falcon tube ("FACS tube") in a volume of 300µl/tube. Such volume was completed with PBS containing 0.09% NaN₃ and 0.5 % BSA to reach 2ml (final volume), gently mixed and centrifuged at 540g for 5min; then, the supernatant was removed using a Pasteur pipette without disturbing the cell pellet. This procedure was repeated twice. The final cell concentration was adjusted with PBS with 0.09% NaN₃ and 0.5 % BSA to 5 x 10⁵ cells/µL and around 100 µL (i.e. 10 million cells) of the final cell suspension/sample were used per tube to be stained and measured in the flow cytometer.

*Staining of the sample.* Two 100µl-aliquots of the above processed specimen samples were subsequently stained with the appropriate volumes (saturating concentrations) of monoclonal antibody combinations (tubes) of the T-MRD panel being the subject of the current invention (Table 6). In the first step, appropriate antibodies against surface markers were added: i) CD34 BV421 / CD45RA BV510 / CD6 BV605 / smCD3 BV711 / CD117+CD13+CD33 BV786 / CD16+NKp80 PE / CD5 PerCP-Cy5.5 / CD7 APC / CD45 AlexaFluor 700 / CD38 APCH7 (i.e. antibody combination 1 in Table 6), and ii) CD34 BV421 / CD45RA BV510 / CD8 BV605 / smCD3 BV711 / CD4 BV786 / CD99 FITC / CD16+NKp80 PE / CD5 PerCP-Cy5.5 / CD7 APC / CD45 AlexaFluor 700 / CD2 APCH7 (i.e. antibody combination 2 in Table 6). After the cells and antibody reagents directed against cell surface markers were mixed well, they were incubated for 30 min at RT protected from light. After this incubation, 2 mL of PBS containing 0.09% NaN3 and 0.5 % BSA was added to the cell pellet, mixed well and centrifuged for 5 min at 540g. Then, the supernatant was discarded using a Pasteur pipette or vacuum system without disturbing the cell pellet, and approximately 50µL of residual volume was left in each tube. Next, the cell pellet was resuspended by gentle mixing, and 100µL of Reagent A (fixative containing PFA) of the Fix&Perm^{™} reagent kit (An der Grub, Vienna, Austria) was subsequently added, followed by another incubation for 15 min at RT protected from light. Subsequently, 2 mL of PBS with 0.09% NaN3 and 0.5% BSA was added to the cell pellet, mixed well and centrifuged for 5 min at 540 g. Then, the supernatant was discarded using a Pasteur pipette or vacuum system without disturbing the cell pellet, and approximately 50µL of residual volume was left in each tube; the cell pellet was resuspended by gently mixing and 100µL of Reagent B (permeabilizing solution containing saponin) of the Fix&Perm^{™} kit was subsequently added. After gently mixing, the appropriate volumes of each of the intracellular antibodies: cyCD3 PE-Vio770 to tube 1 and 2 and additionally TdT FITC to tube 1 were added, mixed and incubated for 15 min at RT protected from light. Afterward, 2 mL of PBS with 0.09% NaN3 and 0.5% BSA was added to the cell pellet, mixed well and centrifuged for 5 min at 540g, the supernatant was discarded using a Pasteur pipette or vacuum system without disturbing the cell pellet, and approximately 50pL residual volume was left in each tube. Upon mixing well the residual volume, the cell pellet was resuspended in 200µL PBS with 0.09% NaN3 and 0.5% BSA, and immediately measured in a BD FACS Lyric or BD LSRFortessa X-20 flow cytometers equipped with 3 lasers and 12 fluorescence detectors.

*Data analysis* First, total BM events displayed on the CD45 vs. SSC dot plot are gated to only include the events with heterogeneous CD45 expression and low side scatter (SSC) values. Then, the selected events are displayed on cyCD3 vs. CD7 dot plot, where cyCD3⁺ events are gated along with all events with heterogeneous expression of CD7. Next, the selected events are shown on CD16+NKp80 vs. smCD3 dot plot, where contaminating CD16+NKp80-expressing NK cells can be gated out. Afterwards, on CD 117+CD 13+CD33 vs. smCD3 dot plot, myeloid cells expressing CD 117+CD 13+CD33 are gated out and after exclusion of these, mature smCD3+/CD6+ T-Cells can be gated out on a CD6 vs. smCD3 dot plot. In the next step, within the remaining events, MRD population can be distinguished among mature T-Cells by differential (high) expression of CD38 on CD7 vs. CD38 dot plot. Finally, MRD is cleaned from the remaining mature T-Cells on CD45 vs. CD5 dot plot by selection of events with lower expression of these markers (see Figure 4).
*Determination of marker separation utility.* To determine the utility of the T-MRD panel markers to discriminate the residual blasts from normal mature T-cells or NK-cells in an MRD setting, a marker scoring system based on the canonical analysis (CA) performed with the Infinicyt software was applied. Top 4 markers from each respective comparison with the highest contribution to separation received 3, 2, 1 or 0.5 points from the highest to the lowest rank, respectively. So obtained marker scores were summarized independently for comparisons of blasts vs. T-cells and blasts vs. NK-cells in both panel tubes (see Figure 3).

**Table 8. Representative expression of the T-MRD panel markers on normal T- and NK-Cells and three subtypes of T-ALL/LBL. Marker expression levels were determined arbitrarily based on the median fluorescence intensity (MFI) measure. Compare Figure 2 for marker expression and Figure 3 for marker contribution to separation.**

| * Some markers (typed in bold) contribute individually to the separation between T-ALL/T-LBL blast cells versus normal mature T-cells and NK-cells, while other markers do so as combination of two or more markers (see also text for further explanation). | | | | | |
|---|---|---|---|---|---|
| **Marker** | **T-Cells** | **NK-Cells** | **T-ALL/LBL Cells*** | | |
| | | | **ETP-ALL** | **smCD3-negative non-ETP-ALL** | **smCD3-positive non-ETP-ALL** |
| cyCD3 | high positive | negative / part positive | low or high positive | low or high positive | high positive |
| **smCD3** | **high positive** | **negative** | **negative** | **negative** | **low or high positive** |
| CD7 | low or high positive | high positive | high positive | high positive | high positive |
| CD45 | high positive | high positive | low or high positive | low or high positive | high positive |
| CD5 | high positive | negative | negative or low positive | low or high positive | low or high positive |
| **CD16+NKp80** | **negative or low positive** | **high positive** | **negative** | **negative** | **negative** |
| CD45RA | negative or positive | high positive | low or high positive | negative or positive | negative or positive |
| **CD34** | **negative** | **negative** | **positive** | **negative or positive** | **negative** |
| CD38 | low positive | low or high positive | high positive | low or high positive | low or high positive |
| **CD117+CD13+CD33** | **negative** | **negative** | **positive** | **negative** | **negative** |
| **TdT** | **negative** | **negative** | **low or high positive** | **low or high positive** | **negative or low positive** |
| **CD6** | **high positive** | **negative** | **negative or low positive** | **negative or low positive** | **negative or low positive** |
| **CD99** | **negative or low positive** | **negative or low positive** | **high positive** | **low or high positive** | **low or high positive** |
| CD2 | negative or positive | negative or positive | negative or low positive | negative or positive | positive |
| CD4 | negative or positive | negative | negative | negative or positive | negative or positive |
| CD8 | negative or positive | negative / part positive | negative | negative or positive | negative or positive |

### REFERENCES

1. Muffly, L. Measurable Residual Disease in Acute Lymphoblastic Leukemia: Techniques and Therapeutic Utility. Clin. Adv. Hematol. Oncol. 2022, 20, 419-421.
2. Lato, M.W.; Przysucha, A.; Grosman, S.; Zawitkowska, J.; Lejman, M. The new therapeutic strategies in pediatric t-cell acute lymphoblastic leukemia. Int. J. Mol. Sci. 2021, 22, doi:10.3390/ijms22094502.
3. Van Dongen, J.J.M.; Van Der Velden, V.H.J.; Brüggemann, M.; Orfao, A. Minimal residual disease diagnostics in acute lymphoblastic leukemia: Need for sensitive, fast, and standardized technologies. Blood 2015, 125, 3996-4009, doi:10.1182/blood-2015-03-580027.
4. Starza, I. Della; Chiaretti, S.; De Propris, M.S.; Elia, L.; Cavalli, M.; De Novi, L.A.; Soscia, R.; Messina, M.; Vitale, A.; Guarini, A.; et al. Minimal residual disease in acute lymphoblastic leukemia: Technical and clinical advances. Front. Oncol. 2019, 9, 1-17, doi:10.3389/fonc.2019.00726.
5. Pieters, R.; De Groot-Kruseman, H.; Van Der Velden, V.; Fiocco, M.; Van Den Berg, H.; De Bont, E.; Egeler, R.M.; Hoogerbrugge, P.; Kaspers, G.; Van Der Schoot, E.; et al. Successful therapy reduction and intensification for childhood acute lymphoblastic leukemia based on minimal residual disease monitoring: Study ALL10 from the Dutch Childhood Oncology Group. J. Clin. Oncol. 2016, 34, 2591-2601, doi:10.1200/JCO.2015.64.6364.
6. Pierce, E.; Mautner, B.; Mort, J.; Blewett, A.; Morris, A.; Keng, M.; El Chaer, F. MRD in ALL: Optimization and Innovations. Curr. Hematol. Malig. Rep. 2022, 17, 69-81, doi:10.1007/s11899-022-00664-6.
7. Flores-Montero, J.; Sanoja-Flores, L.; Paiva, B.; Puig, N.; García-Sánchez, O.; Böttcher, S.; Van Der Velden, V.H.J.; Pérez-Morán, J.J.; Vidriales, M.B.; García-Sanz, R.; et al. Next Generation Flow for highly sensitive and standardized detection of minimal residual disease in multiple myeloma. Leukemia 2017, 31, 2094-2103, doi:10.1038/leu.2017.29.
8. Orfao, A.; Ciudad, J.; Lopez-Berges; Lopez, A.; Vidriales, B.; Caballero, M.D.; Valverde, B.; Gonzalez, M.; San Miguel, J.F. Acute lymphoblastic leukemia (ALL): Detection of minimal residual disease (MRD) at flow cytometry. Leuk. Lymphoma 1994, 13, 87-90, doi: 10.3109/10428199409052682.
9. Campana, D.; Pui, C.H. Detection of minimal residual disease in acute leukemia: methodologic advances and clinical significance. Blood 1995, 85, 1416-34.
10. Campana, D.; Coustan-Smith, E. Detection of minimal residual disease in acute leukemia by flow cytometry. Cytometry 1999, 38, 139-152, doi:10.1002/(sici)1097-0320(19990815)38:4<139::aid-cyto1>3.0.co;2-h.
11. Porwit-MacDonald, A.; Björklund, E.; Lucio, P.; Van Lochem, E.G.; Mazur, J.; Parreira, A.; Van Den Beemd, M.W.M.; Van Wering, E.R.; Baars, E.; Gaipa, G.; et al. BIOMED-1 Concerted Action report: Flow cytometric characterization of CD7+ cell subsets in normal bone marrow as a basis for the diagnosis and follow-up of T cell acute lymphoblastic leukemia (T-ALL). Leukemia 2000, 14, 816-825, doi:10.1038/sj.leu.2401741.
12. Dworzak, M.N.; Fröschl, G.; Printz, D.; Mann, G.; Pötschger, U.; Mühlegger, N.; Fritsch, G.; Gadner, H.; Austrian Berlin-Frankfurt-Münster Study Group Prognostic significance and modalities of flow cytometric minimal residual disease detection in childhood acute lymphoblastic leukemia. Blood 2002, 99, 1952-8.
13. Coustan-Smith, E.; Sancho, J.; Behm, F.G.; Hancock, M.L.; Razzouk, B.I.; Ribeiro, R.C.; Rivera, G.K.; Rubnitz, J.E.; Sandlund, J.T.; Pui, C.H.; et al. Prognostic importance of measuring early clearance of leukemic cells by flow cytometry in childhood acute lymphoblastic leukemia. Blood 2002, 100, 52-58, doi:10.1182/blood-2002-01-0006.
14. Björklund, E.; Mazur, J.; Söderhäll, S.; Porwit-MacDonald, A. Flow cytometric follow-up of minimal residual disease in bone marrow gives prognostic information in children with acute lymphoblastic leukemia. Leukemia 2003, 17, 138-148, doi:10.1038/sj.leu.2402736.
15. Vidriales, M.B.; San-Miguel, J.F.; Orfao, A.; Coustan-Smith, E.; Campana, D. Minimal residual disease monitoring by flow cytometry. Best Pract. Res. Clin. Haematol. 2003, 16, 599-612, doi:10.1016/51521-6926(03)00067-7.
16. Dworzak, M.N.; Fröschl, G.; Printz, D.; De Zen, L.; Gaipa, G.; Ratei, R.; Basso, G.; Biondi, A.; Ludwig, W.D.; Gadner, H. CD99 expression in T-lineage ALL: Implications for flow cytometric detection of minimal residual disease. Leukemia 2004, 18, 703-708, doi:10.1038/sj.leu.2403303.
17. Enein, A.A.A.; Rahman, H.A.A.; Sharkawy, N. El; Abd Elhamid, S.M.; Abbas, S.M.A.; Abdelfaatah, R.; Khalil, M.; Fathalla, L.A. Significance of CD99 expression in T-lineage acute lymphoblastic leukemia. Cancer Biomarkers 2016, 17, 117-123, doi:10.3233/CBM-160608.
18. Salari, F.; Shahjahani, M.; Shahrabi, S.; Saki, N. Minimal residual disease in acute lymphoblastic leukemia: optimal methods and clinical relevance, pitfalls and recent approaches. Med. Oncol. 2014, 31, 1-9, doi:10.1007/s12032-014-0266-3.
19. van Wering, E.R.; van Lochem, E.G.; Leenders, M.; van der Sluijs-Gelling, A.J.; Wind, H.; Gratama, J.W.; Kraan, J.; Preijers, F.W.M.B. Three-color flowcytometric analysis of mature and immature hematological malignancies. A guideline of the Dutch Foundation for Immunophenotyping of Hematological Malignancies (SIHON). J. Biol. Regul. Homeost. Agents 2004, 18, 313-326.
20. Denys, B.; Van Der Sluijs-Gelling, A.J.; Homburg, C.; Van Der Schoot, C.E.; De Haas, V.; Philippé, J.; Pieters, R.; Van Dongen, J.J.M.; Van Der Velden, V.H.J. Improved flow cytometric detection of minimal residual disease in childhood acute lymphoblastic leukemia. Leukemia 2013, 27, 635-641, doi:10.1038/leu.2012.231.
21. Wood, B.L. Flow cytometric monitoring of residual disease in acute leukemia. Methods Mol. Biol. 2013, 999, 123-136, doi:10.1007/978-1-62703-357-2_8.
22. Roshal, M.; Fromm, J.R.; Winter, S.; Dunsmore, K.; Wood, B.L. Immaturity associated antigens are lost during induction for T cell lymphoblastic leukemia: implications for minimal residual disease detection. Cytometry B. Clin. Cytom. 2010, 78, 139-146, doi:10.1002/cyto.b.20511.
23. Brammer, J.E.; Saliba, R.M.; Jorgensen, J.L.; Ledesma, C.; Gaballa, S.; Poon, M.; Maziarz, R.T.; Champlin, R.E.; Hosing, C.; Kebriaei, P. Multi-center analysis of the effect of T-cell acute lymphoblastic leukemia subtype and minimal residual disease on allogeneic stem cell transplantation outcomes. Bone Marrow Transplant. 2017, 52, 20-27, doi:10.1038/bmt.2016.194.
24. DiGiuseppe, J.A.; Wood, B.L. Applications of Flow Cytometric Immunophenotyping in the Diagnosis and Posttreatment Monitoring of B and T Lymphoblastic Leukemia/Lymphoma. Cytom. Part B - Clin. Cytom. 2019, 96, 256-265, doi:10.1002/cyto.b.21833.
25. Patel, J.L.; Smith, L.M.; Anderson, J.; Abromowitch, M.; Campana, D.; Jacobsen, J.; Lones, M.A.; Gross, T.G.; Cairo, M.S.; Perkins, S.L. The immunophenotype of T-lymphoblastic lymphoma in children and adolescents: a Children's Oncology Group report. Br. J. Haematol. 2012, 159, 454-461, doi:10.1111/bjh.12042.
26. Tembhare, P.R.; Chatterjee, G.; Khanka, T.; Ghogale, S.; Badrinath, Y.; Deshpande, N.; Panda, D.; Patkar, N. V; Narula, G.; Girase, K.; et al. Eleven-marker 10-color flow cytometric assessment of measurable residual disease for T-cell acute lymphoblastic leukemia using an approach of exclusion. Cytometry B. Clin. Cytom. 2021, 100, 421-433, doi:10.1002/cyto.b.21939.
27. Wang, H.; Zhou, Y.; Huang, X.; Zhang, Y.; Qian, J.; Li, J.; Li, C.; Li, X.; Lou, Y.; Zhu, Q.; et al. Minimal residual disease level determined by flow cytometry provides reliable risk stratification in adults with T-cell acute lymphoblastic leukaemia. Br. J. Haematol. 2021, 193, 1096-1104, doi:10.1111/bjh.17424.
28. Fossat, C.; Roussel, M.; Arnoux, I.; Asnafi, V.; Brouzes, C.; Garnache-Ottou, F.; Jacob, M.C.; Kuhlein, E.; Macintyre-Davi, E.; Plesa, A.; et al. Methodological aspects of minimal residual disease assessment by flow cytometry in acute lymphoblastic leukemia: A french multicenter study. Cytom. Part B - Clin. Cytom. 2015, 88, 21-29, doi:10.1002/cyto.b.21195.
29. Garand, R.; Beldjord, K.; Cavé, H.; Fossat, C.; Arnoux, I.; Asnafi, V.; Bertrand, Y.; Boulland, M.L.; Brouzes, C.; Clappier, E.; et al. Flow cytometry and IG/TCR quantitative PCR for minimal residual disease quantitation in acute lymphoblastic leukemia: A French multicenter prospective study on behalf of the FRALLE, EORTC and GRAALL. Leukemia 2013, 27, 370-376, doi:10.1038/leu.2012.234.
30. Van Dongen, J.J.M.; Lhermitte, L.; Böttcher, S.; Almeida, J.; Van Der Velden, V.H.J.; Flores-Montero, J.; Rawstron, A.; Asnafi, V.; Lécrevisse, Q.; Lucio, P.; et al. EuroFlow antibody panels for standardized n-dimensional flow cytometric immunophenotyping of normal, reactive and malignant leukocytes. Leukemia 2012, 26, 1908-1975, doi:10.1038/leu.2012.120.
31. Kalina, T.; Flores-Montero, J.; Van Der Velden, V.H.J.; Martin-Ayuso, M.; Böttcher, S.; Ritgen, M.; Almeida, J.; Lhermitte, L.; Asnafi, V.; Mendonça, A.; et al. EuroFlow standardization of flow cytometer instrument settings and immunophenotyping protocols. Leukemia 2012, 26, 1986-2010, doi:10.1038/leu.2012.122.

## Claims

1. A reagent composition for the cytometric detection of minimal residual disease (MRD) in T-cell acute lymphoblastic leukemia (T-ALL) and/or (T-cell lymphoblastic lymphoma (T-LBL), the reagent composition comprising a panel of at least four antibodies conjugated to a detectable label, wherein the at least four conjugated antibodies are directed against markers NKp80, CD16, cytoplasmic CD3 (cyCD3) and surface membrane CD3 (smCD3).

2. Reagent composition according to claim 1, wherein the antibodies directed against markers NKp80 and CD16 are conjugated to the same detectable label.

3. Reagent composition according to claim 1 or 2, wherein the panel further comprises a conjugated antibody directed against CD6.

4. Reagent composition according to any one of the preceding claims, wherein the panel further comprises a conjugated antibody directed against CD45RA.

5. Reagent composition according to any one of the preceding claims, wherein the panel further comprises a set of conjugated antibodies directed against the markers CD117, CD13 and CD33, and wherein each antibody within the set is conjugated to a distinct or to the same label.

6. Reagent composition according to any one of the preceding claims, wherein the panel further comprises a conjugated antibody directed against CD45.

7. Reagent composition according to any one of the preceding claims, wherein the panel further comprises conjugated antibodies directed against at least two of the immature T-cell markers CD99, Terminal deoxynucleotidyl transferase (TdT) and CD34, preferably an antibody against CD34 and antibodie(s) against CD99 and/or TdT, more preferably comprising distinct label-conjugated antibodies directed against CD99, TdT and CD34.

8. Reagent composition according to any one of the preceding claims, wherein the panel further comprises distinctly conjugated antibodies against two or more markers selected from the group consisting of CD2, CD5, CD7, CD4 and CD8.

9. Reagent composition according to claim 7 or 8, further comprising a conjugated antibody directed against CD38.

10. Reagent composition according to any one of the preceding claims, wherein the antibodies are conjugated to a fluorochrome.

11. Reagent composition according to any one claims 1-9, wherein the antibodies are conjugated to a metal isotope, preferably selected from the group consisting of 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt and 209Bi.

12. A set of reagent compositions comprising at least two distinct reagent compositions according to any one of claims 1-11.

13. Set of reagent compositions according to claim 12, comprising
- a first reagent composition comprising conjugated antibodies directed against the markers TdT, CD16, NKp80, CD5, cyCD3, CD7, CD45, CD38, CD34, CD45RA, CD6, smCD3, CD117, CD13 and CD33; and
- a second reagent composition comprising conjugated antibodies directed against the markers CD99, CD16, NKp80, CD5, cyCD3, CD7, CD45, CD2, CD34, CD45RA, CD8, smCD3 and CD4.

14. Set of reagent compositions according to claim 13, wherein each reagent composition contains 12 distinct detectable labels and wherein between the reagent compositions the detectable labels are the same.

15. A diagnostic kit for flow cytometric detection of minimal residual disease (MRD) in T-cell acute lymphoblastic leukemia (T-ALL) and/or (T-cell lymphoblastic lymphoma (T-LBL), comprising at least one reagent composition according to claims 1-11, or a set of reagent compositions according to any one of claims 12-14, optionally together with instructions for use, buffer, and/or control samples.

16. A cytometric method for detecting minimal residual disease (MRD) in T-cell acute lymphoblastic leukemia (T-ALL) and/or (T-cell lymphoblastic lymphoma (T-LBL), comprising the steps of:
(i) contacting an aliquot of a biological sample comprising leukocytes with a reagent composition according to any one of claims 1-11, preferably wherein the sample is selected from the group consisting of blood, bone marrow, cerebrospinal fluid, saliva, tears, pleural effusions, peritoneal fluid, bronchoalveolar lavage, thymus, lymph nodes, mediastinal and abdominal masses, and spleen specimen,
(ii) analyzing leukocytes in said aliquot in a flow or mass cytometer;
(iii) gating on cells for expression of the selected markers detected by the antibodies present in the reagent composition; and
(iv) distinguishing between normal and malignant cells, based on the expression profile of the multiple markers, preferably involving multivariate analysis, more preferably principal component analysis (PCA) and/or canonical analysis.

17. Method according to claim 16, for detecting MRD in Early T-cell precursor (ETP) acute lymphoblastic leukemia/lymphoma (ALL/LBL).

## Patentansprüche

1. Reagenzzusammensetzung zum zytometrischen Nachweis einer minimalen Resterkrankung (MRD) bei akuter lymphoblastischer T-Zell-Leukämie (T-ALL) und/oder lymphoblastischem T-Zell-Lymphom (T-LBL), wobei die Reagenzzusammensetzung ein Panel aus mindestens vier Antikörpern umfasst, konjugiert mit einer nachweisbaren Markierung, wobei die mindestens vier konjugierten Antikörper gegen die Marker NKp80, CD 16, zytoplasmatisches CD3 (cyCD3) und membranständiges CD3 (smCD3) gerichtet sind.

2. Reagenzzusammensetzung nach Anspruch 1, wobei die Antikörper gegen die die Marker NKp80 und CD16 gerichteten sind mit derselben nachweisbaren Markierung konjugiert sind.

3. Reagenzzusammensetzung nach Anspruch 1 oder 2, wobei das Panel ferner einen konjugierten Antikörper umfasst, gerichtet gegen CD6 .

4. Reagenzzusammensetzung nach einem der vorstehenden Ansprüche, wobei das Panel ferner einen konjugierten Antikörper umfasst, gerichtet gegen CD45RA.

5. Reagenzzusammensetzung nach einem der vorstehenden Ansprüche, wobei das Panel ferner einen Satz konjugierter Antikörper umfasst, gerichtet gegen die Marker CD117, CD13 und CD33, und wobei jeder Antikörper innerhalb des Satzes mit einer unterschiedlichen oder derselben Markierung konjugiert ist.

6. Reagenzzusammensetzung nach einem der vorstehenden Ansprüche, wobei das Panel ferner einen konjugierten Antikörper umfasst, gerichtet gegen CD45.

7. Reagenzzusammensetzung nach einem der vorstehenden Ansprüche, wobei das Panel ferner konjugierte Antikörper umfasst, gerichtet gegen mindestens zwei der unreifen T-Zell-Marker CD99, Terminale Desoxynukleotidyltransferase (TdT) und CD34, bevorzugt einen Antikörper gegen CD34 und Antikörper gegen CD99 und/oder TdT, bevorzugter umfassend verschiedene Markierungs-konjugierte Antikörper, gerichtet gegen CD99, TdT und CD34.

8. Reagenzzusammensetzung nach einem der vorstehenden Ansprüche, wobei das Panel ferner unterschiedlich konjugierte Antikörper gegen zwei oder mehr Marker umfasst, ausgewählt aus der Gruppe bestehend aus CD2, CD5, CD7, CD4 und CD8.

9. Reagenzzusammensetzung nach Anspruch 7 oder 8, ferner umfassend einen konjugierten Antikörper, gerichtet gegen CD38.

10. Reagenzzusammensetzung nach einem der vorstehenden Ansprüche, wobei die Antikörper mit einem Fluorochrom konjugiert sind.

11. Reagenzzusammensetzung nach einem der Ansprüche 1 bis 9, wobei die Antikörper mit einem Metallisotop konjugiert sind, bevorzugt ausgewählt aus der Gruppe bestehend aus 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt und 209Bi.

12. Satz von Reagenzzusammensetzungen, umfassend mindestens zwei verschiedene Reagenzzusammensetzungen nach einem der Ansprüche 1 bis 11.

13. Satz von Reagenzzusammensetzungen nach Anspruch 12, umfassend eine erste Reagenzzusammensetzung, umfassend konjugierte Antikörper, gerichtet gegen die Marker TdT, CD16, NKp80, CD5, cyCD3, CD7, CD45, CD38, CD34, CD45RA, CD6, smCD3, CD117, CD13 und CD33; und
smCD3, CDll 7, CD13 und CD33; und
eine zweite Reagenzzusammensetzung, umfassend konjugierte Antikörper, gerichtet gegen die Marker CD99, CD16, NKp80, CD5, cyCD3, CD7, CD45, CD2, CD34, CD45RA, CD8, smCD3 und CD4.

14. Satz von Reagenzzusammensetzungen nach Anspruch 13, wobei jede Reagenzzusammensetzung 12 verschiedene detektierbareMarkierungen enthält und wobei die detektierbaren Markierungen zwischen den Reagenzzusammensetzungen die gleichen sind.

15. Diagnostisches Kit zum durchflusszytometrischen Nachweis von minimaler Resterkrankung (MRD) bei akuter lymphoblastischer T-Zell-Leukämie (T-ALL) und/oder lymphoblastischem T-Zell-Lymphom (T-LBL), umfassend mindestens eine Reagenzzusammensetzung nach den Ansprüchen 1 bis 11, oder einen Satz von Reagenzzusammensetzungen nach einem der Ansprüche 12 bis 14, optional zusammen mit einer Gebrauchsanweisung, Puffer und/oder Kontrollproben.

16. Zytometrisches Verfahren zum Nachweisen minimaler Resterkrankung (MRD) bei akuter lymphoblastischer T-Zell-Leukämie (T-ALL) und/oder lymphoblastischem T-Zell-Lymphom (T-LBL), umfassend die Schritte vo9n:
(i) Inkontaktbringen eines Aliquots einer biologischen Probe, umfassend Leukozyten, mit einer Reagenzzusammensetzung nach einem der Ansprüche 1 bis 11, bevorzugt wobei die Probe ausgewählt ist aus der Gruppe bestehend aus Blut, Knochenmark, Liquor cerebrospinalis, Speichel, Tränen, Pleuraergüssen, Peritonealflüssigkeit, bronchoalveolärer Lavage, Thymus, Lymphknoten, mediastinalen und abdominalen Massen und Milzproben,
(ii) Analysieren von Leukozyten in dem Aliquot in einem Durchfluss- oder Massenzytometer;
(iii) Auswählen von Zellen zur Expression der ausgewählten Marker, detektiert durch Antikörper, die in der Reagenzzusammensetzung vorhanden sind; und
(iv) Unterscheiden zwischen normalen und malignen Zellen basierend auf dem Expressionsprofil der mehreren Marker, bevorzugt unter Einbeziehung einer multivariaten Analyse, noch bevorzugter einer Hauptkomponentenanalyse (PCA) und/oder einer kanonischen Analyse.

17. Verfahren nach Anspruch 16 zum Nachweis von MRD bei akuter lymphoblastischer Leukämie/Lymphom (ALL/LBL) vom Typ "Early T-cell precursor" (ETP).

## Revendications

1. Composition de réactifs pour la détection cytométrique de maladie résiduelle mesurable (MRM) dans le cadre d'une leucémie lymphoblastique aiguë à cellules T (T-ALL) et/ou d'un lymphome lymphoblastique à cellules T (T-LBL), la composition de réactifs comprenant un panel d'au moins quatre anticorps conjugués à une étiquette détectable, dans laquelle les au moins quatre anticorps conjugués sont dirigés contre les marqueurs NKp80, CD16, CD3 cytoplasmique (cyCD3) et CD3 de membrane de surface (smCD3).

2. Composition de réactifs selon la revendication 1, dans laquelle les anticorps dirigés contre les marqueurs NKp80 et CD16 sont conjugués à la même étiquette détectable.

3. Composition de réactifs selon la revendication 1 ou 2, dans laquelle le panel comprend en outre un anticorps conjugué dirigé contre CD6.

4. Composition de réactifs selon l'une quelconque des revendications précédentes, dans laquelle le panel comprend en outre un anticorps conjugué dirigé contre CD45RA.

5. Composition de réactifs selon l'une quelconque des revendications précédentes, dans laquelle le panel comprend en outre un jeu d'anticorps conjugués dirigés contre les marqueurs CD117, CD13 et CD33, et dans laquelle chaque anticorps de ce jeu est conjugué à une étiquette distincte ou à la même étiquette.

6. Composition de réactifs selon l'une quelconque des revendications précédentes, dans laquelle le panel comprend en outre un anticorps conjugué dirigé contre CD45.

7. Composition de réactifs selon l'une quelconque des revendications précédentes, dans laquelle le panel comprend en outre des anticorps conjugués dirigés contre au moins deux parmi les marqueurs de cellules T immatures CD99, désoxynucléotidyle transférase terminale (TdT) et CD34, de préférence un anticorps dirigé contre CD34 et un ou plusieurs anticorps dirigés contre CD99 et/ou TdT, mieux encore comprend des anticorps conjugués à des étiquettes distinctes et dirigés contre CD99, TdT et CD34.

8. Composition de réactifs selon l'une quelconque des revendications précédentes, dans laquelle le panel comprend en outre des anticorps distinctement conjugués dirigés contre deux marqueurs ou plus choisis dans le groupe constitué par CD2, CD5, CD7, CD4 et CD8.

9. Composition de réactifs selon la revendication 7 ou 8, comprenant en outre un anticorps conjugué dirigé contre CD38.

10. Composition de réactifs selon l'une quelconque des revendications précédentes, dans laquelle les anticorps sont conjugués à un fluorochrome.

11. Composition de réactifs selon l'une quelconque des revendications 1 à 9, dans laquelle les anticorps sont conjugués à un isotope métallique, de préférence choisi dans le groupe constitué par 89Y, 106Cd, 110Cd, 111Cd, 112Cd, 113Cd, 114Cd, 115In, 116Cd, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 194Pt, 195Pt, 196Pt, 197Au, 198Pt et 209Bi.

12. Jeu de compositions de réactifs comprenant au moins deux compositions de réactifs distinctes selon l'une quelconque des revendications 1 à 11.

13. Jeu de compositions de réactifs selon la revendication 12, comprenant
- une première composition de réactifs comprenant des anticorps conjugués dirigés contre les marqueurs TdT, CD16, NKp80, CD5, cyCD3, CD7, CD45, CD38, CD34, CD45RA, CD6, smCD3, CD117, CD13 et CD33 ; et
- une deuxième composition de réactifs comprenant des anticorps conjugués dirigés contre les marqueurs CD99, CD16, NKp80, CD5, cyCD3, CD7, CD45, CD2, CD34, CD45RA, CD8, smCD3 et CD4.

14. Jeu de compositions de réactifs selon la revendication 13, dans lequel chaque composition de réactifs contient 12 étiquettes détectables distinctes et dans lequel, parmi les compositions de réactifs, les étiquettes détectables sont les mêmes.

15. Trousse de diagnostic pour une détection par cytométrie en flux de maladie résiduelle mesurable (MRM) dans le cadre d'une leucémie lymphoblastique aiguë à cellules T (T-ALL) et/ou d'un lymphome lymphoblastique à cellules T (T-LBL), comprenant au moins une composition de réactifs selon les revendications 1 à 11, ou un jeu de compositions de réactifs selon l'une quelconque des revendications 12 à 14, éventuellement conjointement avec des instructions d'utilisation, un tampon, et/ou des échantillons témoins.

16. Méthode de cytométrie pour détecter une maladie résiduelle mesurable (MRM) dans le cadre d'une leucémie lymphoblastique aiguë à cellules T (T-ALL) et/ou d'un lymphome lymphoblastique à cellules T (T-LBL), comprenant les étapes de :
(i) mise en contact d'une aliquote d'un échantillon biologique comprenant des leucocytes avec une composition de réactifs selon l'une quelconque des revendications 1 à 11, de préférence dans laquelle l'échantillon est choisi dans le groupe constitué par les échantillons de sang, moelle osseuse, liquide céphalo-rachidien, salive, larmes, effusions pleurales, liquide péritonéal, lavage broncho-alvéolaire, thymus, ganglions lymphatiques, masses médiastinales et abdominales, et rate,
(ii) analyse des leucocytes dans ladite aliquote dans un cytomètre en flux ou en masse ;
(iii) sélection de cellules en fonction de l'expression des marqueurs sélectionnés détectés par les anticorps présents dans la composition de réactifs ; et
(iv) distinction entre les cellules normales et malignes, sur la base du profil d'expression des multiples marqueurs, de préférence impliquant une analyse multivariée, mieux encore une analyse en composantes principales (PCA) et/ou une analyse canonique.

17. Méthode selon la revendication 16 pour détecter une MRM dans le cadre d'un lymphome/d'une leucémie lymphoblastique aiguë (ALL/LBL) à précurseurs de cellules T précoces (ETP).
